# EUROPEAN PATENT APPLICATION

(11) **EP 4 368 200 A2**
(43) Date of publication of application: **15.05.2024**
(21) Application number: 24157330.2
(22) Date of filing: 04.04.2018
(51) Int. Cl.: A61K 39/00

(54) **ANTICANCER COMBINATION THERAPY**

(30) Priority: 05.04.2017 EP 17165111; 24.10.2017 EP 17197930
(62) Divisional of application: 18715685.6
(71) Applicant: Boehringer Ingelheim International GmbH, 55216 Ingelheim am Rhein (DE)
(72) Inventor: RUDOLPH, Dorothea, Ingrid, 55216 INGELHEIM AM RHEIN (DE); RESCHKE, Markus, 55216 INGELHEIM AM RHEIN (DE)
(74) Representative: Lutze, Oliver

(57) **Abstract**

The invention describes anti-cancer therapies comprising using an MDM2 inhibitor in combination with a PD-1 antagonist and a LAG-3 antagonist, each as described herein.

## Description

The tumor suppressor protein p53 is a sequence specific transcription factor and plays a central role in the regulation of several cellular processes, including cell cycle and growth arrest, apoptosis, DNA repair, senescence, angiogenesis, and innate immunity. The Mouse Double Minute 2 (MDM2) protein (or its human homolog also known as HDM2) acts to down-regulate p53 activity in an auto-regulatory manner, and under normal cellular conditions (absence of stress), the MDM2 protein serves to maintain p53 activity at low levels. MDM2 directly inhibits the transactivation function of p53, exports p53 out of the nucleus, and promotes proteasome-mediated degradation of p53 through its E3 ubiquitin ligase activity.

Deregulation of the MDM2/p53 balance by overexpression of MDM2 or by p53 mutation or loss leads to malignant transformation of normal cells. Presently p53 is known to play a key role in practically all types of human cancers, and the mutation or loss of the p53 gene can be identified in more than 50 % of all human cancers worldwide. Analysis of 28 different types of human cancers in nearly 4,000 human tumor samples showed that MDM2 is amplified in 7 % of human cancers and that MDM2 overexpression by amplification and p53 mutations are largely mutually exclusive (Momand et al., Nucleic Acid Res (1998) 26:3453-3459).

Because of the powerful tumor suppressor function of p53, reactivation of p53 has been long sought as a potentially novel cancer therapeutic strategy. In tumor harboring wild-type p53, MDM2 is the primary cellular inhibitor of p53 activity, and overexpression of MDM2 was found in many human tumors. Since MDM2 inhibits p53 through a direct protein-protein interaction, blocking this interaction using small molecules was pursued in several academic and industrial pharmaceutical laboratories in the last decade. A variety of non-peptide, drug-like small molecule as *e.g.* imidazole compounds (*e.g*. Nutlins or RG7112), benzodiazepinedione compounds, spirooxindole compounds (*e.g*. MI-219), substituted piperidines, pyrrolidinone compounds (*e.g*. PXN820-dl) and modifications thereof have been selected and designed in order to block MDM2/p53 interaction as a means to reactivate p53 in cells (Vassilev et al., Science (2004) 303:844-848; Grasberger et al., J Med Chem (2005) 48:909-912; Parks et al., Bioorg Med Chem Lett (2005) 15:765; Ding et al., J Am Soc (2005) 127:10130-10131; WO 2010/028862, US Patent 7,884,107, WO 2008/119741). A number of potent MDM2/p53 inhibitors have been evaluated in animal models of human cancer for their anti-tumor activity (Vassilev et al., Science (2004) 303:844-848; Tovar et al, Cancer Res (2013) 73 (8): 2587 - 2597; Ding et al , Journal of Medicinal Chemistry (2013) 56 (14): 5979 - 5983; Rew et al, Journal of Medicinal Chemistry (2012) 55: 4936 - 4954; Sun et al, Journal of Medicinal Chemistry (2014) 57 (4): 1454 - 1472).

In the pediatric preclinical testing program (PPTP) of the NCI, early evidence for high level anti-proliferative activity of RG7112, an inhibitor of the MDM2-p53 interaction, could be observed *in vitro and in vivo.* In particular, RG-7112 showed cytotoxic activity with lower median IC₅₀ values for p53 wild-type vs. p53 mutant cell lines (Carol et al., Pediatric Blood and Cancer (2013) 60(4):633-641). Moreover, RG-7112 induced tumor growth inhibition in solid tumor xenograft models and was particularly efficacious in in acute lymphoblastic leukemia (ALL) xenograft models with mixed-lineage leukemia (MLL) rearrangement, (Carol et al., Pediatric Blood and Cancer (2013) 60(4):633-641). Additionally, the antiproliferative and proapoptotic activity of RG7112 has been observed in human acute myeloid leukemia (AML) and human prostate tumor xenograft models harboring p53 wild-type (Tovar et al, Cancer Res (2013) 73 (8): 2587 - 2597).

Cancer immunotherapy is a branch of oncology in which the immune system is used to treat cancer which is in stark contrast to existing common methods of treatment in which the tumour is directly excised or treated. This therapeutic concept is based on the identification of a number of proteins on the surface of T-cells which act to inhibit the immune function of these cells. Listed among these proteins is PD-1.

PD-1 (Programmed cell Death 1) is a cell surface receptor protein expressed on T-cells. The protein functions as an "immune checkpoint" inhibitor, *i.e.* it acts to modulate the activity of cells in the immune system so as to regulate and limit autoimmune diseases. It has been recently understood that many cancers can protect themselves from the immune system by modifying "immune checkpoint" inhibitors and thus avoid detection.

PD-1 has two ligands, PD-L1 and PD-L2, which interact with the cell surface receptor. On binding, PD-1 induces an intracellular signal which negatively regulates T-cell response.

As detailed above, PD-1 is a key regulator of T-cell activity. Recently, it has been shown in a range of different cancer settings that the antagonistic PD-1 antibody molecules nivolumab and pembrolizumab can be used to stimulate the immune system and thereby treat cancer.

Lymphocyte Activation Gene-3 (LAG-3; CD223) is a type I transmembrane protein mainly expressed on the cell surface of activated T cells but also found on subsets of NK and dendritic cells. LAG3 is closely related to CD4, which is a co-receptor for T helper cell activation. Both molecules have four extracellular Ig-like domains and require binding to their ligand, major histocompatibility complex (MHC) class II, for their functional activity. On binding to MHC-II, LAG3 induces an intracellular signal which negatively regulates T-cell response. Recent studies have revealed that LAG-3 and PD-1 are co-expressed on tumor infiltrating lymphocytes (TILs) suggesting that they may contribute to tumor-mediated immune suppression. It is thought that chronic exposure to antigens leads to a progressive inactivation of T cells through a process termed "exhaustion". Exhausted T cells often co-express negative regulatory receptors such as PD-1 and LAG-3.

Despite encouraging clinical results of PD-1 antagonistic monoclonal antibodies nivolumab and pembrolizumab, up to 70 % of treated patients do not respond to treatment. Preclinical data with patient-derived T cells as well as from syngeneic tumour mouse models have demonstrated that tumour-derived T cells frequently express other inhibitory receptors in addition to PD1. Combined neutralization of PD-1 and LAG-3 using antagonistic monoclonal antibody molecules increased reactivation of T cells and improved tumour rejection compared to PD-1 neutralization alone, in *in vitro* and *in vivo* models. Based on these results, it is expected that neutralization of LAG-3 will enhance the efficacy of antagonistic PD-1 mAbs.

The efficacy of therapeutic agents can be improved by using combination therapies (in particular in oncology) with other compounds and/or improving the dosage schedule. Even if the concept of combining several therapeutic agents has already been suggested, and although various combination therapies are under investigation and in clinical trials, there is still a need for new and efficient therapeutic concepts for the treatment of cancer diseases, *e.g.* solid tumors, which show advantages over standard therapies, such as for example better treatment outcome, beneficial effects, superior efficacy and/or improved tolerability, such as *e.g.* reduced side effects of the combined treatment. Specifically, there is a need for additional treatment options for patients with cancers like, *e.g.,* lung cancer (*e.g.* NSCLC), brain cancer (*e.g*. glioblastoma, also including brain metastasis of cancers with other origin), soft tissue sarcoma (*e.g*. liposarcoma) and genitourinary cancer (*e.g*. bladder cancer).

It is thus an object of the present invention to provide combination treatments/methods of combination treatment providing certain advantages compared to treatments/methods of treatment currently used and/or known in the prior art. These advantages may include *in vivo* efficacy (*e.g*. improved clinical response, extend of the response, increase of the rate of response, duration of response, disease stabilization rate, duration of stabilization, time to disease progression, progression free survival (PFS) and/or overall survival (OS), later occurence of resistance and the like), safe and well tolerated administration and reduced frequency and severity of adverse events.

In this context, the inventors of the present application, surprisingly, discovered that the use of an inhibitor of the interaction between MDM2 and p53 (referred to herein as "MDM2 inhibitor") in combination with a PD-1 (Programmed cell Death 1) antagonist, *i.e.* preferably an *anti*-PD-1 or *anti*-PD-L1 antibody in the context of the invention, and with a LAG-3 (Lymphocyte Activation Gene-3) antagonist, *i.e.* preferably an *anti*-LAG-3 antibody, has the potential to improve clinical outcome compared to the use of either an MDM2 inhibitor, a PD-1 antagonist or a LAG-3 antagonist alone or any dual combination of these therapeutic agents.

Thus, the invention relates to methods for the treatment and/or prevention of oncological or hyperproliferative diseases, in particular cancer, as described herein, comprising the combined administration of an MDM2 inhibitor, a PD-1 antagonist and a LAG-3 antagonist, each as described herein, as well as to medical uses, to uses, to pharmaceutical compositions or combinations and kits comprising such therapeutic agents.

Further, the invention relates to *anti*-cancer therapies comprising using an MDM2 inhibitor, a PD-1 antagonist and a LAG-3 antagonist, each as described herein, in combination.

For the treatment of diseases of oncological nature, a large number of anticancer agents (including target-specific and non-target-specific anticancer agents) have already been suggested, which can be used as monotherapy or as combination therapy involving more than one agent (*e.g*. dual or triple combination therapy) and/or which may be combined with radiotherapy (*e.g*. irradiation treatment), radio-immunotherapy and/or surgery.

It is a purpose of the present invention to provide combination therapies with the therapeutic agents described herein for treating or controlling various malignancies (*e.g*. based on cooperative, complementary, interactive or improving effects of the active components involved in combination).

Thus, in one aspect the invention provides a method of treating and/or preventing an oncological or hyperproliferative disease, in particular cancer, comprising administering to a patient in need thereof a therapeutically effective amount of an MDM2 inhibitor, a therapeutically effective amount of a PD-1 antagonist and a therapeutically effective amount of a LAG-3 antagonist, each as described herein.

In another aspect the method of treating and/or preventing further comprises administering a therapeutically effective amount of one or more additional therapeutic agent(s) as described herein.

Such a combined treatment may be given as a non-fixed (*e.g*. free) combination of the substances or in the form of a fixed combination, including kit-of-parts.

In another aspect the invention provides a combination of an MDM2 inhibitor, a PD-1 antagonist and a LAG-3 antagonist, each as described herein, particularly for use in a method of treating and/or preventing an oncological or hyperproliferative disease, in particular cancer, as described herein, said method comprising administering to a patient in need thereof a therapeutically effective amount of the combination.

In another aspect the combination further comprises one or more additional therapeutic agent(s) as described herein.

In another aspect the invention refers to an MDM2 inhibitor as described herein for use in a method of treating and/or preventing an oncological or hyperproliferative disease, in particular cancer, as described herein, said method comprising administering the MDM2 inhibitor in combination with a PD-1 antagonist and a LAG-3 antagonist, each as described herein, to a patient in need thereof.

In another aspect the method of treating and/or preventing further comprises administering in combination with one or more additional therapeutic agent(s) as described herein.

In another aspect the invention refers to a PD-1 antagonist as described herein for use in a method of treating and/or preventing an oncological or hyperproliferative disease, in particular cancer, as described herein, said method comprising administering the PD-1 antagonist in combination with an MDM2 inhibitor and a LAG-3 antagonist, each as described herein, to a patient in need thereof.

In another aspect the method of treating and/or preventing further comprises administering in combination with one or more additional therapeutic agent(s) as described herein.

In another aspect the invention refers to a LAG-3 antagonist as described herein for use in a method of treating and/or preventing an oncological or hyperproliferative disease, in particular cancer, as described herein, said method comprising administering the LAG-3 antagonist in combination with an MDM2 inhibitor and a PD-1 antagonist, each as described herein, to a patient in need thereof.

In another aspect the method of treating and/or preventing further comprises administering in combination with one or more additional therapeutic agent(s) as described herein.

In another aspect the invention refers to a kit comprising
- a first pharmaceutical composition or dosage form comprising an MDM2 inhibitor as described herein, and, optionally, one or more pharmaceutically acceptable carriers, excipients and/or vehicles,
- a second pharmaceutical composition or dosage form comprising a PD-1 antagonist as described herein, and, optionally, one or more pharmaceutically acceptable carriers, excipients and/or vehicles, and
- a third pharmaceutical composition or dosage form comprising a LAG-3 antagonist as described herein, and, optionally, one or more pharmaceutically acceptable carriers, excipients and/or vehicles.

In another aspect the kit comprises one or more additional pharmaceutical composition(s) or dosage form(s) each comprising one additional therapeutic agent as described herein, and, optionally, one or more pharmaceutically acceptable carriers, excipients and/or vehicles.

In another aspect the invention refers to the aforementioned kits further comprising
• a package insert comprising printed instructions for simultaneous, concurrent, sequential, successive, alternate or separate use in the treatment and/or prevention of an oncological or hyperproliferative disease, in particular cancer, as described herein, in a patient in need thereof.

In another aspect the invention refers to the aforementioned kits for use in a method of treating and/or preventing an oncological or hyperproliferative disease, in particular cancer, as described herein.

In another aspect the invention refers to a pharmaceutical composition comprising
- an MDM2 inhibitor as described herein,
- a PD-1 antagonist as described herein,
- a LAG-3 antagonist as described herein, and
- optionally, one or more pharmaceutically acceptable carriers, excipients and/or vehicles.

In another aspect the pharmaceutical composition comprises one or more additional therapeutic agent(s) as described herein.

In another aspect the invention refers to the use of an MDM2 inhibitor as described herein for preparing a pharmaceutical composition for use in a method of treating and/or preventing an oncological or hyperproliferative disease, in particular cancer, as described herein, wherein the MDM2 inhibitor is to be used in combination with a PD-1 antagonist and a LAG-3 antagonist, each as described herein.

In another aspect of the use of the MDM2 inhibitor the MDM2 inhibitor is to be used in combination with a PD-1 antagonist and a LAG-3 antagonist, each as described herein, and one or more additional therapeutic agent(s) as described herein.

In another aspect the invention refers to the use of a PD-1 antagonist as described herein for preparing a pharmaceutical composition for use in a method of treating and/or preventing an oncological or hyperproliferative disease, in particular cancer, as described herein, wherein the PD-1 antagonist is to be used in combination with an MDM2 inhibitor and a LAG-3 antagonist, each as described herein.

In another aspect of the use of the PD-1 antagonist the PD-1 antagonist is to be used in combination with an MDM2 inhibitor and a LAG-3 antagonist, each as described herein, and one or more additional therapeutic agent(s) as described herein.

In another aspect the invention refers to the use of a LAG-3 antagonist as described herein for preparing a pharmaceutical composition for use in a method of treating and/or preventing an oncological or hyperproliferative disease, in particular cancer, as described herein, wherein the LAG-3 antagonist is to be used in combination with an MDM2 inhibitor and a PD-1 antagonist, each as described herein.

In another aspect of the use of the LAG-3 antagonist the LAG-3 antagonist is to be used in combination with an MDM2 inhibitor and a PD-1 antagonist, each as described herein, and one or more additional therapeutic agent(s) as described herein.

In another aspect the invention refers to the use of an MDM2 inhibitor, a PD-1 antagonist and a LAG-3 antagonist, each as described herein, for preparing a pharmaceutical composition for use in a method of treating and/or preventing an oncological or hyperproliferative disease, in particular cancer, as described herein.

In another aspect the invention refers to the use of an MDM2 inhibitor, a PD-1 antagonist, a LAG-3 antagonist and one or more additional therapeutic agent(s), each as described herein, for preparing a pharmaceutical composition for use in a method of treating and/or preventing an oncological or hyperproliferative disease, in particular cancer, as described herein.

In another aspect the invention refers to a combination, a pharmaceutical composition or a kit according to the invention, each as described herein, comprising, consisting or consisting essentially of an MDM2 inhibitor, a PD-1 antagonist and a LAG-3 antagonist, each as described herein, for use in a method of treating and/or preventing an oncological or hyperproliferative disease, in particular cancer, as described herein.

The invention also discloses the use of an inhibitor of the interaction between MDM2 and p53 (referred to herein as "MDM2 inhibitor") in combination with a PD-1 (Programmed cell Death 1) antagonist, *i.e.* preferably an *anti*-PD-1 or *anti*-PD-L1 antibody or with a LAG-3 (Lymphocyte Activation Gene-3) antagonist, *i.e.* preferably an *anti*-LAG-3 antibody. The MDM2 inhibitor is preferably any compound disclosed in the embodiments A1-A47, the PD-1 antagonist is any compound disclosed in the embodiments B1-B15 and the LAG-3 antagonist is any compound disclosed in the embodiments C1-C12.

The invention also discloses methods for the treatment and/or prevention of oncological or hyperproliferative diseases, in particular cancer, as described herein, comprising the combined administration of an MDM2 inhibitor and a PD-1 antagonist or an MDM2 inhibitor and a LAG-3 antagonist, each as described herein, as well as to medical uses, to uses, to pharmaceutical compositions or combinations and kits comprising such therapeutic agents.

Further, the invention also discloses *anti*-cancer therapies comprising using an MDM2 inhibitor in combination with a PD-1 antagonist or an MDM2 inhibitor in combination with a LAG-3 antagonist.

### Brief description of the figures

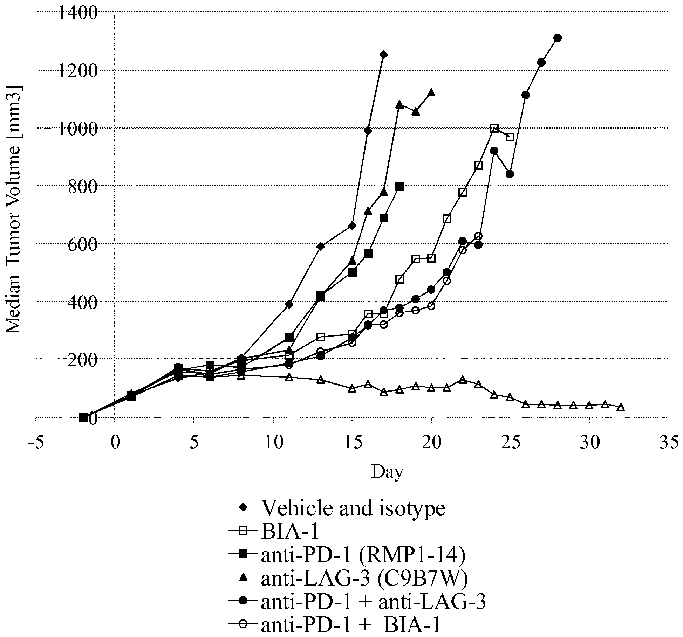

Figure 1 shows the anti-tumor activity of the exemplary MDM2 inhibitor BIA-1 as single agent or in combination with RMP1-14, a tool antibody to mouse PD-1, or in combination with RMP1-14 and C9B7W, a tool antibody to mouse LAG-3, compared to vehicle/isotype or RMP1-14 single agent or C9B7W single agent or a combination of RMP1-14 and C9B7W in a subcutaneous syngeneic mouse model derived from the melanoma cell line B16-F10 in C57BL/6 mice.

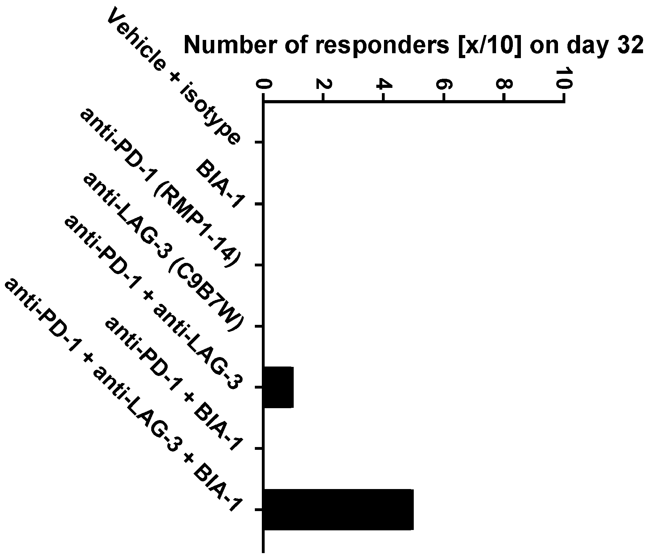

Figure 2 shows the number of responders, defined as the number of animals with a tumor that has a relative tumor volume < 1 on day 32 compared to treatment start on day 1, for each treatment group of the study shown in Figure 1.

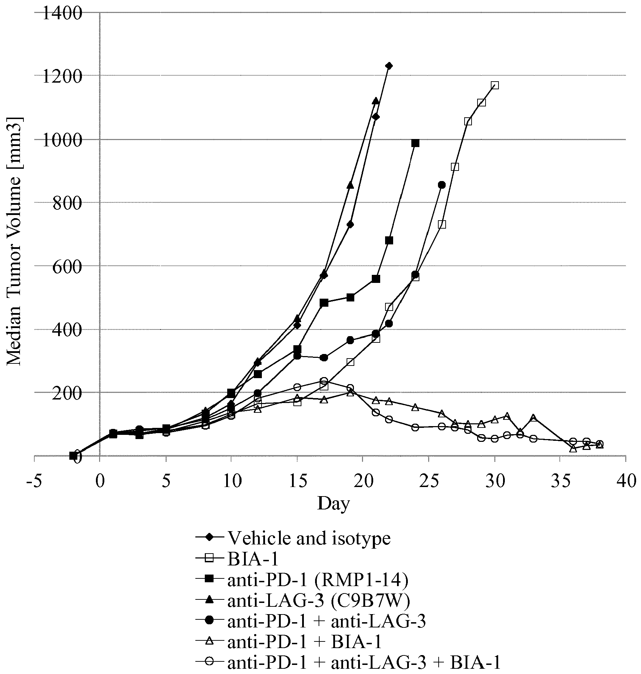

Figure 3 shows the anti-tumor activity of the exemplary MDM2 inhibitor BIA-1 as single agent or in combination with RMP1-14, a tool antibody to mouse PD-1, or in combination with RMP1-14 and C9B7W, a tool antibody to mouse LAG-3, compared to vehicle/isotype or RMP1-14 single agent or C9B7W single agent or a combination of RMP1-14 and C9B7W in a subcutaneous syngeneic mouse model derived from the colon cancer cell line Colon26 in Balb/C mice.

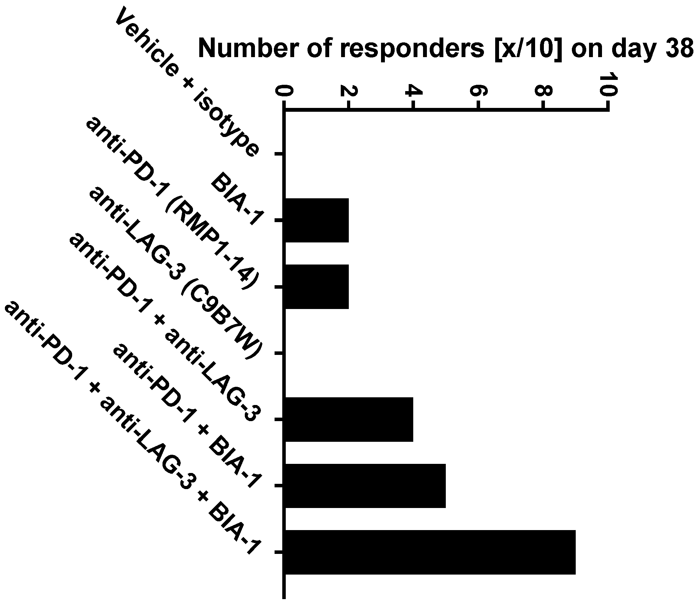

Figure 4 shows the number of responders, defined as the number of animals with a tumor that has a relative tumor volume < 1 on day 38 compared to treatment start on day 1, for each treatment group of the study shown in Figure 3.

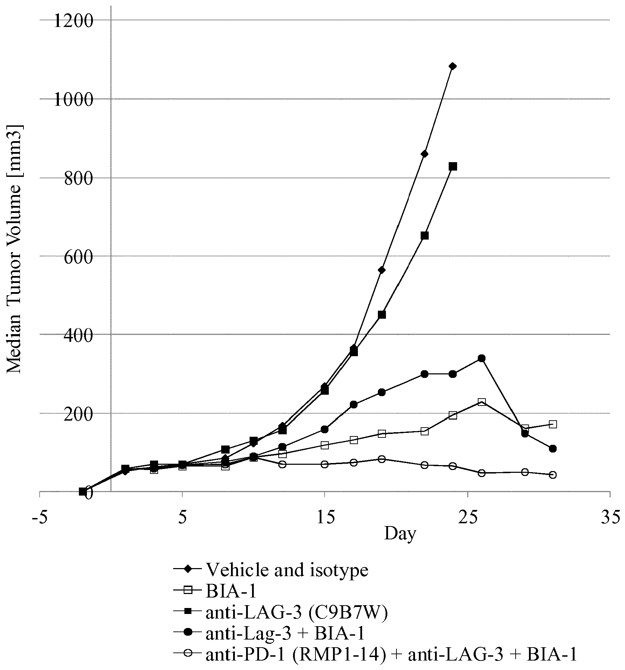

Figure 5 shows the anti-tumor activity of the exemplary MDM2 inhibitor BIA-1 as single agent or in combination with C9B7W, a tool antibody to mouse LAG-3, or in combination with RMP1-14, a tool antibody to mouse PD-1, and C9B7W compared to vehicle/isotype or C9B7W single agent in a subcutaneous syngeneic mouse model derived from the colon cancer cell line Colon26 in Balb/C mice.

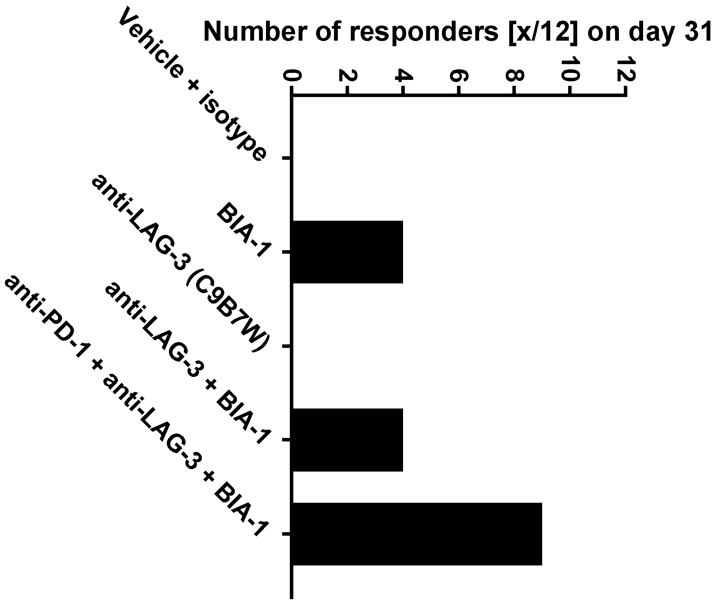

Figure 6 shows the number of responders, defined as the number of animals with a tumor that has a relative tumor volume < 1 on day 31 compared to treatment start on day 1, for each treatment group of the study shown in Figure 5.

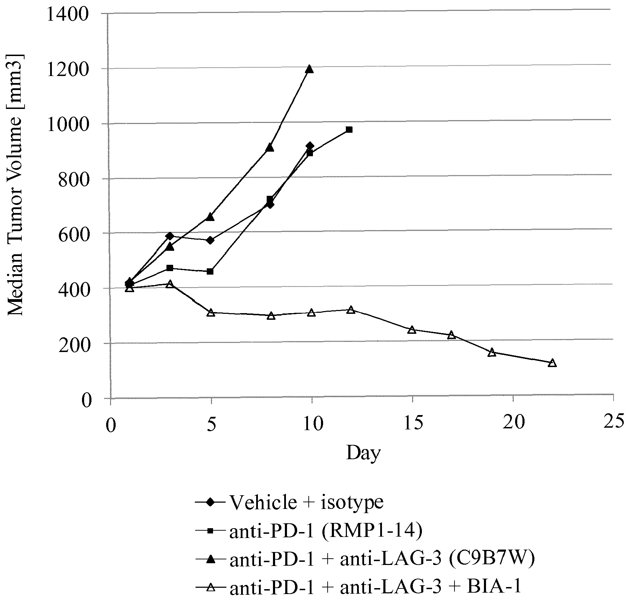

Figure 7: ASB-XIV tumor bearing mice were pretreated for 8 days with RMP1-14, a tool antibody to mouse PD-1, and those showing no tumor-growth control were randomized at a tumor volume of about 400 mm³ for follow-up treatment receiving either the exemplary MDM2 inhibitor BIA-1 in combination with RMP1-14 and C9B7W, a tool antibody to mouse LAG-3, or vehicle/isotype, RMP1-14 single agent or the combination of RMP1-14 and C9B7W

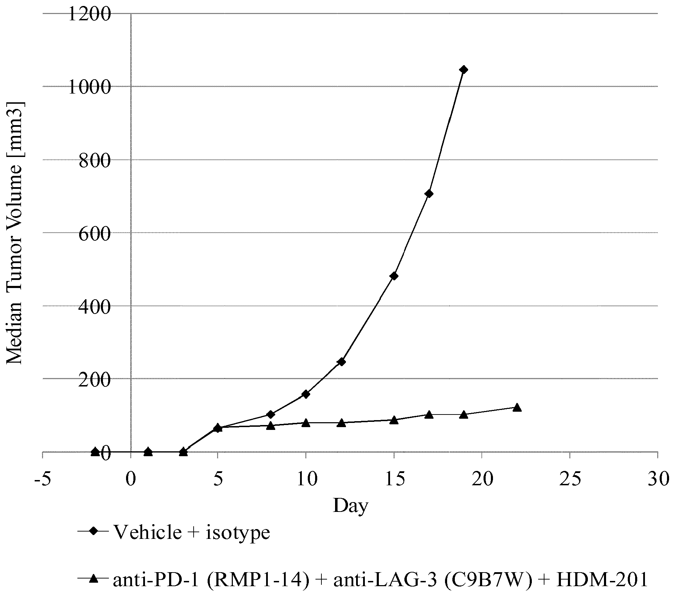

Figure 8 shows the anti-tumor activity of the MDM2 inhibitor HDM-201 in combination with RMP1-14, a tool antibody to mouse PD-1, and C9B/W, a tool antibody to mouse LAG-3 in a subcutaneous syngeneic mouse model derived from the colon cancer cell line Colon26 in Balb/C mice.

### MDM2 inhibitor

The MDM2 inhibitor within the meaning of this invention and all its embodiments is a compound which inhibits the interaction of MDM2 and p53.

Preferably, the MDM2 inhibitor within this invention and all its embodiments is selected from the group consisting of the following **(A0):**
• an MDM2 inhibitor (*i.e*. a compound) as (generically and/or specifically) disclosed in WO 2015/155332, or a pharmaceutically acceptable salt thereof;
• an MDM2 inhibitor (*i.e*. a compound) as (generically and/or specifically) disclosed in WO 2016/001376, or a pharmaceutically acceptable salt thereof;
• an MDM2 inhibitor (*i.e*. a compound) as (generically and/or specifically) disclosed in WO 2016/026937, or a pharmaceutically acceptable salt thereof;
• an MDM2 inhibitor (*i.e.* a compound) as (generically and/or specifically) disclosed in WO 2017/060431, or a pharmaceutically acceptable salt thereof;
• an MDM2 inhibitor (*i.e*. a compound) as (generically and/or specifically) disclosed in WO 2013/111105, or a pharmaceutically acceptable salt thereof;
• an MDM2 inhibitor (*i.e.* a compound) as (generically and/or specifically) disclosed in WO 2011/076786, or a pharmaceutically acceptable salt thereof;
• HDM-201, *i.e.* Example 102 in WO 2013/111105 (page 207), or a pharmaceutically acceptable salt thereof;
• NVP-CGM097, *i.e.* Example 106 in WO 2011/076786 (page 265), or a pharmaceutically acceptable salt thereof;
• the MDM2 inhibitor known as RG-7112, or a pharmaceutically acceptable salt thereof;
• the MDM2 inhibitor known as MK-8242, or a pharmaceutically acceptable salt thereof;
• the MDM2 inhibitor known as RG-7388, or a pharmaceutically acceptable salt thereof;
• the MDM2 inhibitor known as SAR405838, or a pharmaceutically acceptable salt thereof;
• the MDM2 inhibitor known as AMG-232, or a pharmaceutically acceptable salt thereof;
• the MDM2 inhibitor known as DS-3032, or a pharmaceutically acceptable salt thereof;
• the MDM2 inhibitor known as RG-7775, or a pharmaceutically acceptable salt thereof;
• the MDM2 inhibitor known as APG-115, or a pharmaceutically acceptable salt thereof;
• any one of the MDM2 inhibitors 1 to 36 in Table 1 as disclosed in WO 2017/060431, or a pharmaceutically acceptable salt thereof:

**Table 1**

| | |
|---|---|
| **1** | 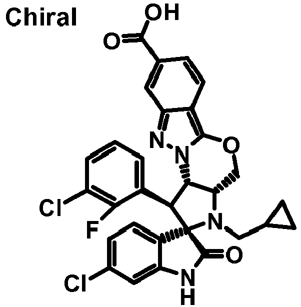 |
| **2** | 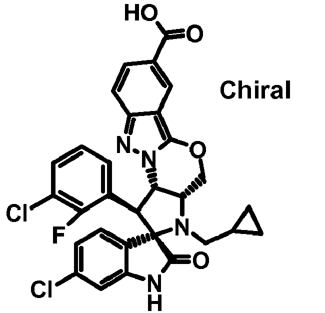 |
| **3** | 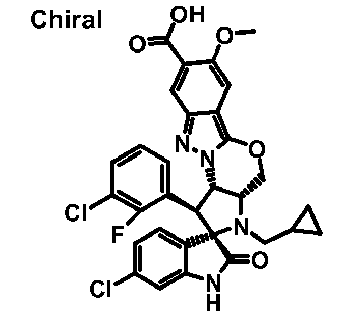 |
| **4** | 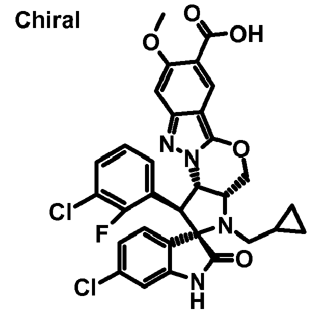 |
| **5** | 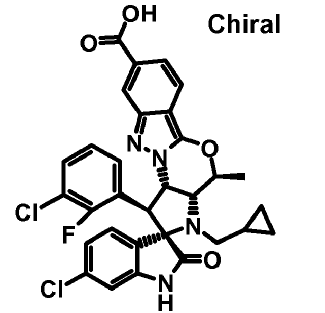 |
| **6** | 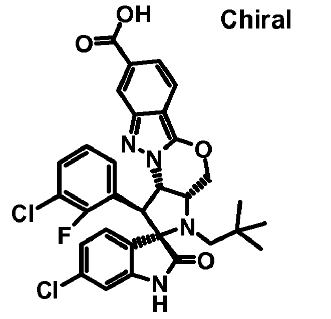 |
| **7** | 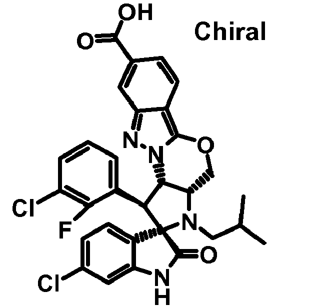 |
| **8** | 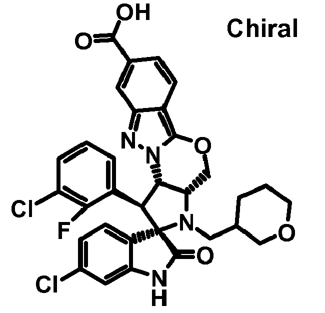 |
| **9** | 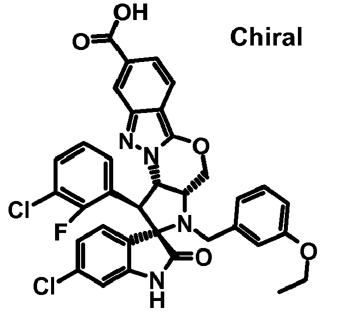 |
| **10** | 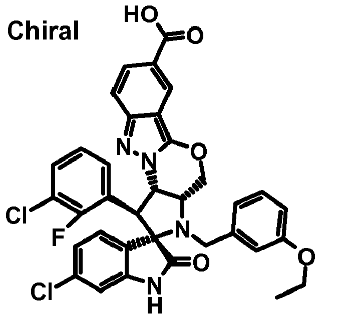 |
| **11** | 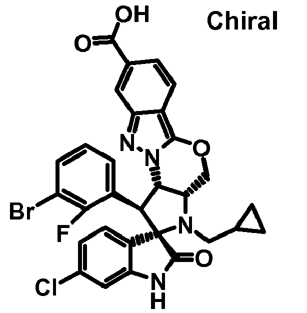 |
| **12** | 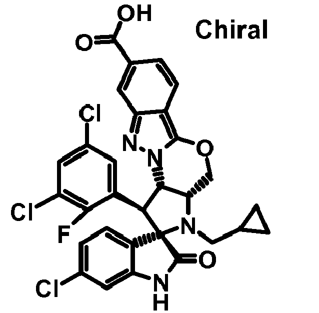 |
| **13** | 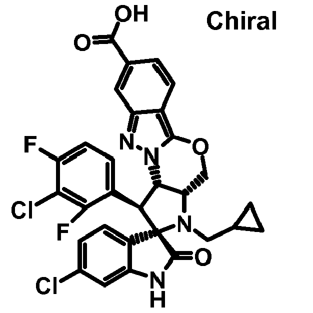 |
| **14** | 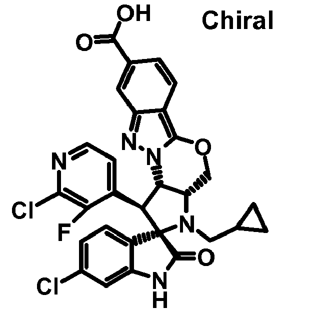 |
| **15** | 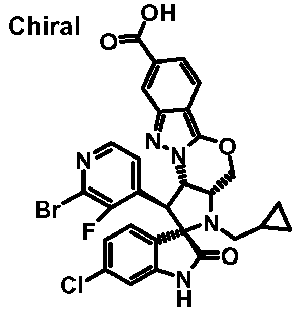 |
| **16** | 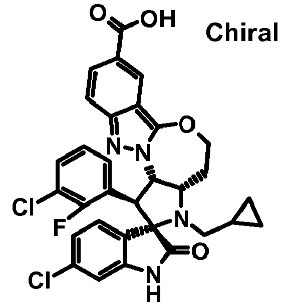 |
| **17** | 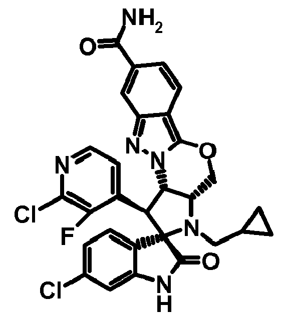 |
| **18** | 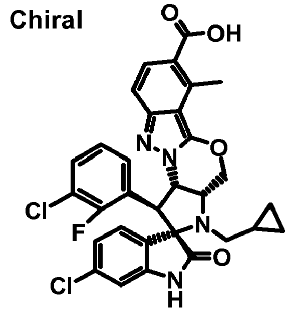 |
| **19** | 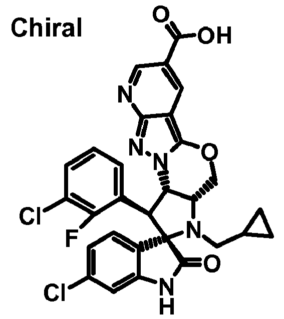 |
| **20** | 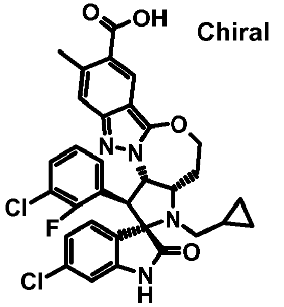 |
| **21** | 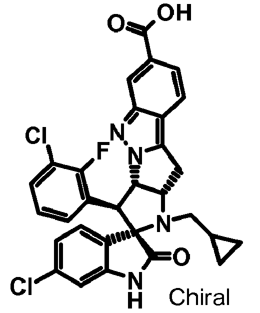 |
| **22** | 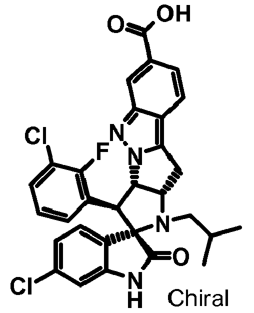 |
| **23** | 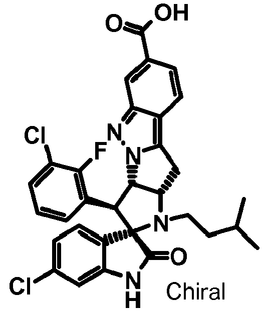 |
| **24** | 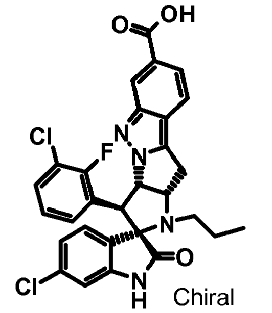 |
| **25** | 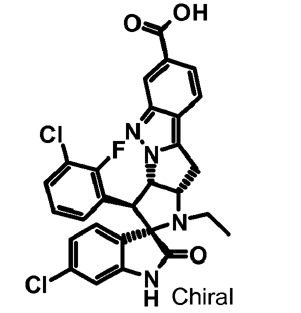 |
| **26** | 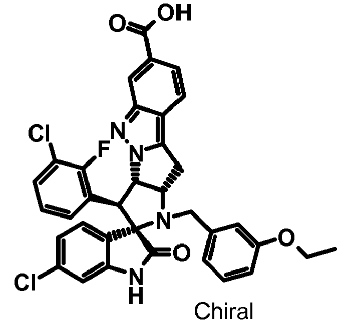 |
| **27** | 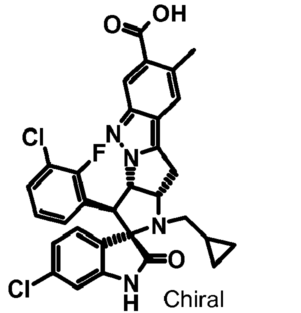 |
| **28** | 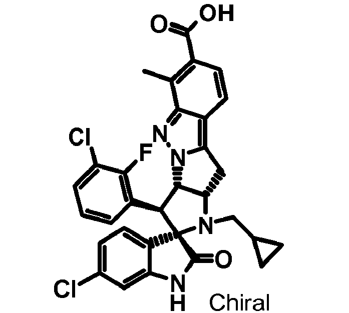 |
| **29** | 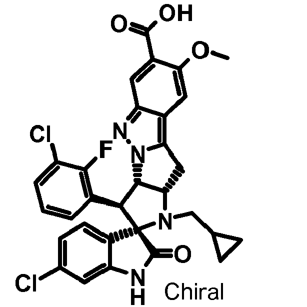 |
| **30** | 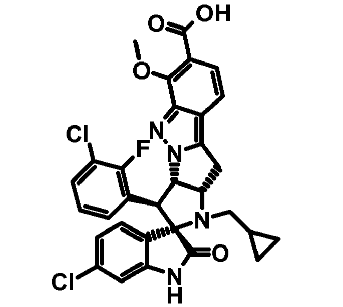 |
| **31** | 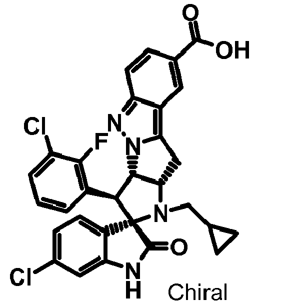 |
| **32** | 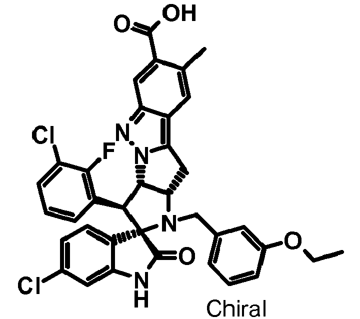 |
| **33** | 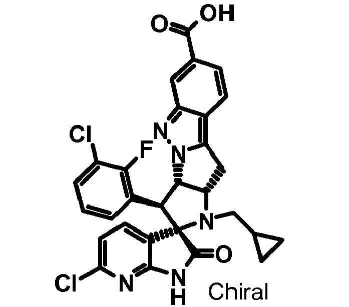 |
| **34** | 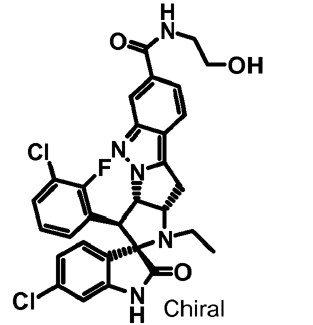 |
| **35** | 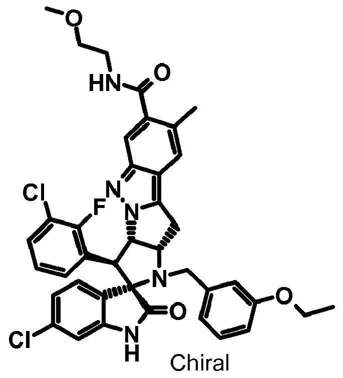 |
| **36** | 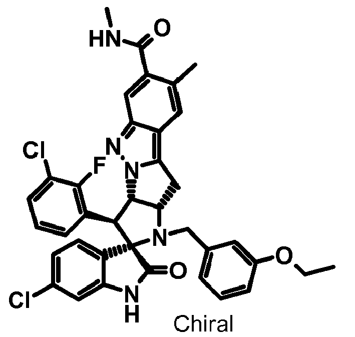 |

The term "MDM2 inhibitor" as used herein also includes the MDM2 inhibitors listed above in the form of a tautomer, of a pharmaceutically acceptable salt, of a hydrate or of a solvate (including a hydrate or solvate of a pharmaceutically acceptable salt). It also includes the MDM2 inhibitor in all its solid, preferably crystalline, forms and in all the crystalline forms of its pharmaceutically acceptable salts, hydrates and solvates (including hydrates and solvates of pharmaceutically acceptable salts).

All MDM2 inhibitors listed above are known in the art with the respective synthesis and properties. All patent applications referred to above are incorporated by reference in their entirety.

In one embodiment the MDM2 inhibitor is HDM-201 or a pharmaceutically acceptable salt thereof **(A1).**

In another embodiment the MDM2 inhibitor is NVP-CGM097 or a pharmaceutically acceptable salt thereof **(A2).**

In another embodiment the MDM2 inhibitor is compound 1 in table 1 or a pharmaceutically acceptable salt thereof **(A3).**

In another embodiment the MDM2 inhibitor is compound 2 in table 1 or a pharmaceutically acceptable salt thereof **(A4).**

In another embodiment the MDM2 inhibitor is compound 3 in table 1 or a pharmaceutically acceptable salt thereof **(A5).**

In another embodiment the MDM2 inhibitor is compound 4 in table 1 or a pharmaceutically acceptable salt thereof **(A6).**

In another embodiment the MDM2 inhibitor is compound 5 in table 1 or a pharmaceutically acceptable salt thereof **(A7).**

In another embodiment the MDM2 inhibitor is compound 6 in table 1 or a pharmaceutically acceptable salt thereof **(A8).**

In another embodiment the MDM2 inhibitor is compound 7 in table 1 or a pharmaceutically acceptable salt thereof **(A9).**

In another embodiment the MDM2 inhibitor is compound 8 in table 1 or a pharmaceutically acceptable salt thereof **(A10).**

In another embodiment the MDM2 inhibitor is compound 9 in table 1 or a pharmaceutically acceptable salt thereof **(A11**).

In another embodiment the MDM2 inhibitor is compound 10 in table 1 or a pharmaceutically acceptable salt thereof **(A12).**

In another embodiment the MDM2 inhibitor is compound 11 in table 1 or a pharmaceutically acceptable salt thereof **(A13).**

In another embodiment the MDM2 inhibitor is compound 12 in table 1 or a pharmaceutically acceptable salt thereof **(A14).**

In another embodiment the MDM2 inhibitor is compound 13 in table 1 or a pharmaceutically acceptable salt thereof **(A15).**

In another embodiment the MDM2 inhibitor is compound 14 in table 1 or a pharmaceutically acceptable salt thereof **(A16).**

In another embodiment the MDM2 inhibitor is compound 15 in table 1 or a pharmaceutically acceptable salt thereof **(A17).**

In another embodiment the MDM2 inhibitor is compound 16 in table 1 or a pharmaceutically acceptable salt thereof **(A18).**

In another embodiment the MDM2 inhibitor is compound 17 in table 1 or a pharmaceutically acceptable salt thereof **(A19).**

In another embodiment the MDM2 inhibitor is compound 18 in table 1 or a pharmaceutically acceptable salt thereof **(A20).**

In another embodiment the MDM2 inhibitor is compound 19 in table 1 or a pharmaceutically acceptable salt thereof **(A21).**

In another embodiment the MDM2 inhibitor is compound 20 in table 1 or a pharmaceutically acceptable salt thereof **(A22).**

In another embodiment the MDM2 inhibitor is compound 21 in table 1 or a pharmaceutically acceptable salt thereof **(A23).**

In another embodiment the MDM2 inhibitor is compound 22 in table 1 or a pharmaceutically acceptable salt thereof **(A24).**

In another embodiment the MDM2 inhibitor is compound 23 in table 1 or a pharmaceutically acceptable salt thereof **(A25).**

In another embodiment the MDM2 inhibitor is compound 24 in table 1 or a pharmaceutically acceptable salt thereof **(A26).**

In another embodiment the MDM2 inhibitor is compound 25 in table 1 or a pharmaceutically acceptable salt thereof **(A27).**

In another embodiment the MDM2 inhibitor is compound 26 in table 1 or a pharmaceutically acceptable salt thereof **(A28).**

In another embodiment the MDM2 inhibitor is compound 27 in table 1 or a pharmaceutically acceptable salt thereof **(A29).**

In another embodiment the MDM2 inhibitor is compound 28 in table 1 or a pharmaceutically acceptable salt thereof **(A30).**

In another embodiment the MDM2 inhibitor is compound 29 in table 1 or a pharmaceutically acceptable salt thereof **(A31).**

In another embodiment the MDM2 inhibitor is compound 30 in table 1 or a pharmaceutically acceptable salt thereof **(A32).**

In another embodiment the MDM2 inhibitor is compound 31 in table 1 or a pharmaceutically acceptable salt thereof **(A33).**

In another embodiment the MDM2 inhibitor is compound 32 in table 1 or a pharmaceutically acceptable salt thereof **(A34).**

In another embodiment the MDM2 inhibitor is compound 33 in table 1 or a pharmaceutically acceptable salt thereof **(A35).**

In another embodiment the MDM2 inhibitor is compound 34 in table 1 or a pharmaceutically acceptable salt thereof **(A36).**

In another embodiment the MDM2 inhibitor is compound 35 in table 1 or a pharmaceutically acceptable salt thereof **(A37).**

In another embodiment the MDM2 inhibitor is compound 36 in table 1 or a pharmaceutically acceptable salt thereof **(A38).**

In another embodiment the MDM2 inhibitor is RG-7112 or a pharmaceutically acceptable salt thereof **(A39).**

In another embodiment the MDM2 inhibitor is MK-8242 or a pharmaceutically acceptable salt thereof **(A40).**

In another embodiment the MDM2 inhibitor is RG-7388 or a pharmaceutically acceptable salt thereof **(A41).**

In another embodiment the MDM2 inhibitor is RG-7388 or a pharmaceutically acceptable salt thereof **(A42).**

In another embodiment the MDM2 inhibitor is SAR405838 or a pharmaceutically acceptable salt thereof **(A43).**

In another embodiment the MDM2 inhibitor is AMG-232 or a pharmaceutically acceptable salt thereof **(A44).**

In another embodiment the MDM2 inhibitor is DS-3032 or a pharmaceutically acceptable salt thereof **(A45).**

In another embodiment the MDM2 inhibitor is RG-7775 or a pharmaceutically acceptable salt thereof **(A46).**

In another embodiment the MDM2 inhibitor is APG-115 or a pharmaceutically acceptable salt thereof **(A47).**

All embodiments **(A1)** to **(A47)** are preferred embodiments of embodiment **(A0)** in respect of the nature of the MDM2 inhibitor.

To be used in therapy, the MDM2 inhibitor is included into pharmaceutical compositions appropriate to facilitate administration to animals or humans.

Typical pharmaceutical compositions for administering the MDM2 inhibitor of the invention include for example tablets, capsules, suppositories, solutions, e.g. solutions for injection (s.c., i.v., i.m.) and infusion, elixirs, emulsions or dispersible powders. The content of the pharmaceutically active compound(s) may be in the range from 0.1 to 90 wt.-%, preferably 40 to 60 wt.-% of the composition as a whole, *e.g.* in amounts which are sufficient to achieve the desired dosage range. The single dosages may, if necessary, be given several times a day to deliver the desired total daily dose.

Typical tablets may be obtained, for example, by mixing the active substance(s), optionally in combination, with known excipients, for example inert diluents such as calcium carbonate, calcium phosphate, cellulose or lactose, disintegrants such as corn starch or alginic acid or crospovidon, binders such as starch or gelatine, lubricants such as magnesium stearate or talc and/or agents for delaying release, such as carboxymethyl cellulose, cellulose acetate phthalate, or polyvinyl acetate. The tablets may be prepared by usual processes, such as *e.g*. by direct compression or roller compaction. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

Syrups or elixirs containing the active substance(s) may additionally contain a sweetener such as saccharine, cyclamate, glycerol or sugar and a flavour enhancer, e.g. a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection and infusion are prepared in the usual way, e.g. with the addition of isotonic agents, preservatives such as p-hydroxybenzoates, or stabilisers such as alkali metal salts of ethylenediamine tetraacetic acid, optionally using emulsifiers and/or dispersants, whilst if water is used as the diluent, for example, organic solvents may optionally be used as solvating agents or dissolving aids, and transferred into injection vials or ampoules or infusion bottles.

Capsules containing the active substance(s) may for example be prepared by mixing the active substance(s) with inert carriers such as lactose or sorbitol and packing them into gelatine capsules.

Typical suppositories may be made for example by mixing the active substance(s) with carriers provided for this purpose, such as neutral fats or polyethyleneglycol or the derivatives thereof.

Excipients which may be used include, for example, water, pharmaceutically acceptable organic solvents such as paraffins (*e.g.* petroleum fractions), vegetable oils (*e.g*. groundnut or sesame oil), mono- or polyfunctional alcohols (*e.g*. ethanol or glycerol), carriers such as *e.g*. natural mineral powders (*e.g*. kaolins, clays, talc, chalk), synthetic mineral powders (*e.g*. highly dispersed silicic acid and silicates), sugars (*e.g*. cane sugar, lactose and glucose) emulsifiers (*e.g*. lignin, spent sulphite liquors, methylcellulose, starch and polyvinylpyrrolidone) and lubricants (*e.g*. magnesium stearate, talc, stearic acid and sodium lauryl sulphate).

The MDM2 inhibitor of this invention and all its embodiments is administered by the usual methods, preferably by oral or parenteral route, most preferably by oral route. For oral administration the tablets may contain, apart from the abovementioned carriers, additives such as sodium citrate, calcium carbonate and dicalcium phosphate together with various additives such as starch, preferably potato starch, gelatine and the like. Moreover, lubricants such as magnesium stearate, sodium lauryl sulphate and talc may be used at the same time for the tabletting process. In the case of aqueous suspensions the active substances may be combined with various flavour enhancers or colourings in addition to the excipients mentioned above.

For parenteral use, solutions of the active substances with suitable liquid carriers may be used.

The dosage for oral use for MDM2 inhibitors, in particular for MDM2 inhibitors in table 1, is from 1 mg to 2000 mg per dose (*e.g*. 10 mg to 1000 mg per dose; in a more preferred embodiment from 10 mg to 500 mg per dose; most preferred is from 10 mg to 100 mg per dose). All amounts given refer to the free base of the MDM2 inhibitor and may be proportionally higher if a pharmaceutically acceptable salt or other solid form is used.

In one embodiment the MDM2 inhibitor, in particular an MDM2 inhibitor in table 1, is dosed once daily (q.d.).

In one embodiment the MDM2 inhibitor, in particular an MDM2 inhibitor in table 1, is dosed on day 1 and day 8 of a 28 day-cycle.

In one embodiment the MDM2 inhibitor, in particular an MDM2 inhibitor in table 1, is dosed on day 1 of a 28 day-cycle.

In one embodiment the MDM2 inhibitor is dosed on day 1 to day 7 of a 28 day-cycle.

The dosage for intravenous use is from 1 mg to 1000 mg per hour, preferably between 5 and 500 mg per hour.

However, it may sometimes be necessary to depart from the amounts specified, depending on the body weight, the route of administration, the individual response to the drug, the nature of its formulation and the time or interval over which the drug is administered. Thus, in some cases it may be sufficient to use less than the minimum dose given above, whereas in other cases the upper limit may have to be exceeded. When administering large amounts it may be advisable to divide them up into a number of smaller doses spread over the day.

### PD-1 antagonist

A PD-1 antagonist within the meaning of this invention and all of its embodiments is a compound that inhibits the interaction of PD-1 with its receptor(s), preferably an *anti*-PD-1 antibody or an *anti*-PD-L1 antibody.

Preferably, the PD-1 antagonist, *i.e.* the anti-PD-1 antibody or *anti*-PD-L1 antibody within this invention and all its embodiments is a humanized or fully human *anti*-PD-1 antibody or a humanized or fully human *anti*-PD-L1 antibody.

More preferably, the PD-1 antagonist within this invention and all its embodiments is selected from the group consisting of the following **(B0):**
- pembrolizumab;
- nivolumab;
- pidilizumab;
- atezolizumab;
- avelumab;
- durvalumab;
- an anti-PD-1 antibody (generically and/or specifically) disclosed in WO 2015/112900:
   ∘ any one of the antibodies as defined in table 1 in WO 2015/112900 (page 171)
   ∘ any one of the humanized antibodies as defined in table 1 in WO 2015/112900 (page 171)
   ∘ any one of BAP049-hum01 to BAP049-hum16 as defined in table 1 in WO 2015/112900 (page 171)
   ∘ any one of BAP049-Clone-A to BAP049-Clone-E as defined in table 1 in WO 2015/112900 (page 171)
- PDR-001;
- an *anti*-PD-L1 antibody (generically and/or specifically) disclosed in WO 2016/061142:
   ∘ any one of the antibodies as defined in table 1 in WO 2016/061142 (page 265);
   ∘ any one of the humanized antibodies as defined in table 1 in WO 2016/061142 (page 265);
   ∘ any one of BAP058-hum01 to BAP058-hum17 as defined in table 1 in WO 2016/061142 (page 265)
   ∘ any one of BAP058-Clone-K to BAP058-Clone-O as defined in table 1 in WO 2016/061142 (page 265)
- an anti-PD-1 antibody PD1-1, PD1-2, PD1-3, PD1-4, and PD1-5 as disclosed herein below.

Pembrolizumab (formerly also known as lambrolizumab; trade name Keytruda; also known as MK-3475) disclosed e.g. in Hamid, O. et al. (2013) New England Journal of Medicine 369(2):134-44, is a humanized IgG4 monoclonal antibody that binds to PD-1; it contains a mutation at C228P designed to prevent Fc-mediated cytotoxicity. Pembrolizumab is e.g. disclosed in US 8,354,509 and WO2009/114335. It is approved by the FDA for the treatment of patients suffering from unresectable or metastatic melanoma and patients with metastatic NSCLC.

Nivolumab (CAS Registry Number: 946414-94-4; BMS-936558 or MDX1106b) is a fully human IgG4 monoclonal antibody which specifically blocks PD-1, lacking detectable antibody-dependent cellular toxicity (ADCC). Nivolumab is e.g. disclosed in US 8,008,449 and WO2006/121168. It has been approved by the FDA for the treatment of patients suffering from unresectable or metastatic melanoma, metastatic NSCLC and advanced renal cell carcinoma.

Pidilizumab (CT-011; Cure Tech) is a humanized IgG1k monoclonal antibody that binds to PD-1. Pidilizumab is e.g. disclosed in WO 2009/101611.

Atezolizumab (Tecentriq, also known as MPDL3280A) is a phage-derived human IgG1k monoclonal antibody targeting PD-L1 and is described e.g. in Deng et al. mAbs 2016;8:593-603. It has been approved by the FDA for the treatment of patients suffering from urothelial carcinoma.

Avelumab is a fully human anti-PD-L1 IgG1 monoclonal antibody and described in e.g. Boyerinas et al. Cancer Immunol. Res. 2015;3:1148-1157.

Durvalumab (MEDI4736) is a human IgG1k monoclonal antibody with high specificity to PD-L1 and described in e.g. Stewart et al. Cancer Immunol. Res. 2015;3:1052-1062 or in Ibrahim et al. Semin. Oncol. 2015;42:474-483.

PDR-001 or PDR001 is a high-affinity, ligand-blocking, humanized anti-PD-1 IgG4 antibody that blocks the binding of PD-L1 and PD-L2 to PD-1. PDR-001 is disclosed in WO2015/112900 and WO2017/019896.

Antibodies PD1-1 to PD1-5 are antibody molecules defined by the sequences as shown in Table 2, wherein HC denotes the (full length) heavy chain and LC denotes the (full length) light chain:

**Table 2**

| **SEQ ID NO:** | **Sequence name** | **Amino acid sequence** |
|---|---|---|
| 1 | HC of PD1-1 | EVMLVESGGGLVQPGGSLRLSCTASGFTFSASAMSWVRQ APGKGLEWVAYISGGGGDTYYSSSVKGRFTISRDNAKNSL YLQMNSLRAEDTAVYYCARHSNVNYYAMDYWGQGTLVTV SSASTKGPSVFPLAPCSRSTSESTAALGCLVKDYFPEPVTV SWNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKSL SLSLG |
| 2 | LC of PD1-1 | EIVLTQSPATLSLSPGERATMSCRASENIDTSGISFMNWYQ QKPGQAPKLLIYVASNQGSGIPARFSGSGSGTDFTLTISRLE PEDFAVYYCQQSKEVPWTFGQGTKLEIKRTVAAPSVFIFPP SDEQLKSGTASVVCLLNNFYPREAKVQWKVDNALQSGNS QESVTEQDSKDSTYSLSSTLTLSKADYEKHKVYACEVTHQ GLSSPVTKSFNRGEC |
| 3 | HC of PD1-2 | EVMLVESGGGLVQPGGSLRLSCTASGFTFSASAMSWVRQ APGKGLEWVAYISGGGGDTYYSSSVKGRFTISRDNAKNSL YLQMNSLRAEDTAVYYCARHSNPNYYAMDYWGQGTLVTV SSASTKGPSVFPLAPCSRSTSESTAALGCLVKDYFPEPVTV SWNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKSL SLSLG |
| 4 | LC of PD1-2 | EIVLTQSPATLSLSPGERATMSCRASENIDTSGISFMNWYQ QKPGQAPKLLIYVASNQGSGIPARFSGSGSGTDFTLTISRLE PEDFAVYYCQQSKEVPWTFGQGTKLEIKRTVAAPSVFIFPP SDEQLKSGTASVVCLLNNFYPREAKVQWKVDNALQSGNS QESVTEQDSKDSTYSLSSTLTLSKADYEKHKVYACEVTHQ GLSSPVTKSFNRGEC |
| 5 | HC of PD1-3 | EVMLVESGGGLVQPGGSLRLSCTASGFTFSKSAMSWVRQ APGKGLEWVAYISGGGGDTYYSSSVKGRFTISRDNAKNSL YLQMNSLRAEDTAVYYCARHSNVNYYAMDYWGQGTLVTV SSASTKGPSVFPLAPCSRSTSESTAALGCLVKDYFPEPVTV SWNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKSL SLSLG |
| 6 | LC of PD1-3 | EIVLTQSPATLSLSPGERATMSCRASENIDVSGISFMNWYQ QKPGQAPKLLIYVASNQGSGIPARFSGSGSGTDFTLTISRLE PEDFAVYYCQQSKEVPWTFGQGTKLEIKRTVAAPSVFIFPP SDEQLKSGTASVVCLLNNFYPREAKVQWKVDNALQSGNS QESVTEQDSKDSTYSLSSTLTLSKADYEKHKVYACEVTHQ GLSSPVTKSFNRGEC |
| 7 | HC of PD1-4 | EVMLVESGGGLVQPGGSLRLSCTASGFTFSKSAMSWVRQ APGKGLEWVAYISGGGGDTYYSSSVKGRFTISRDNAKNSL YLQMNSLRAEDTAVYYCARHSNVNYYAMDYWGQGTLVTV SSASTKGPSVFPLAPCSRSTSESTAALGCLVKDYFPEPVTV SWNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKSL SLSLG |
| 8 | LC of PD1-4 | EIVLTQSPATLSLSPGERATMSCRASENIDVSGISFMNWYQ QKPGQAPKLLIYVASNQGSGIPARFSGSGSGTDFTLTISRLE PEDFAVYYCQQSKEVPWTFGQGTKLEIKRTVAAPSVFIFPP SDEQLKSGTASVVCLLNNFYPREAKVQWKVDNALQSGNS QESVTEQDSKDSTYSLSSTLTLSKADYEKHKVYACEVTHQ GLSSPVTKSFNRGEC |
| 9 | HC of PD1-5 | EVMLVESGGGLVQPGGSLRLSCTASGFTFSKSAMSWVRQ APGKGLEWVAYISGGGGDTYYSSSVKGRFTISRDNAKNSL YLQMNSLRAEDTAVYYCARHSNVNYYAMDYWGQGTLVTV SSASTKGPSVFPLAPCSRSTSESTAALGCLVKDYFPEPVTV SWNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKSL SLSLG |
| 10 | LC of PD1-5 | EIVLTQSPATLSLSPGERATMSCRASENIDVSGISFMNWYQ QKPGQAPKLLIYVASNQGSGIPARFSGSGSGTDFTLTISRLE PEDFAVYYCQQSKEVPWTFGQGTKLEIKRTVAAPSVFIFPP SDEQLKSGTASVVCLLNNFYPREAKVQWKVDNALQSGNS QESVTEQDSKDSTYSLSSTLTLSKADYEKHKVYACEVTHQ GLSSPVTKSFNRGEC |

Specifically, the anti-PD-1 antibody molecule described herein above has:
(PD1-1) a heavy chain comprising the amino acid sequence of SEQ ID NO:1 and a light chain comprising the amino acid sequence of SEQ ID NO:2; or
(PD1-2) a heavy chain comprising the amino acid sequence of SEQ ID NO:3 and a light chain comprising the amino acid sequence of SEQ ID NO:4; or
(PD1-3) a heavy chain comprising the amino acid sequence of SEQ ID NO:5 and a light chain comprising the amino acid sequence of SEQ ID NO:6; or
(PD1-4) a heavy chain comprising the amino acid sequence of SEQ ID NO:7 and a light chain comprising the amino acid sequence of SEQ ID NO:8; or
(PD1-5) a heavy chain comprising the amino acid sequence of SEQ ID NO:9 and a light chain comprising the amino acid sequence of SEQ ID NO:10.

The INNs as used herein are also meant to encompass all biosimilar antibodies having the same, or substantially the same, amino acid sequences as the originator antibody, including but not limited to those biosimilar antibodies authorized under 42 USC §262 subsection (k) in the US and equivalent regulations in other jurisdictions.

All PD-1 antagonists listed above are known in the art with their respective manufacture, therapeutic use and properties. All patent applications referred to above are incorporated by reference in their entirety.

In one embodiment the PD-1 antagonist is pembrolizumab **(B1).**

In another embodiment the PD-1 antagonist is nivolumab **(B2).**

In another embodiment the PD-1 antagonist is pidilizumab **(B3).**

In another embodiment the PD-1 antagonist is atezolizumab **(B4).**

In another embodiment the PD-1 antagonist is avelumab **(B5).**

In another embodiment the PD-1 antagonist is durvalumab **(B6).**

In another embodiment the PD-1 antagonist is PDR-001 **(B7).**

In another embodiment the PD-1 antagonist is BAP049-Clone-B as defined in table 1 in WO 2015/112900 (page 171) **(B8).**

In another embodiment the PD-1 antagonist is BAP049-Clone-E as defined in table 1 in WO 2015/112900 (page 171) **(B9).**

In another embodiment the PD-1 antagonist is selected from the group consisting of BAP058-Clone-K to BAP058-Clone-O as defined in table 1 in WO 2016/061142 (page 265) **(B10).**

In another embodiment the PD-1 antagonist is PD1-1 **(B11).**

In another embodiment the PD-1 antagonist is PD1-2 **(B12).**

In another embodiment the PD-1 antagonist is PD1-3 **(B13).**

In another embodiment the PD-1 antagonist is PD1-4 **(B14).**

In another embodiment the PD-1 antagonist is PD1-5 **(B15).** All embodiments **(B1)** to **(B15)** are preferred embodiments of embodiment **(B0)** in respect of the nature of the PD-1 antagonist.

In one embodiment BAP049-Clone-E as defined in table 1 in WO 2015/112900 (page 171) is dosed and administered according to the schedules disclosed in WO 2017/019896 (page 336, last paragraph)

For a more detailed description for PD-1 antagonists already marketed and their use it is referred to the respective Summary of Product Characteristics (incorporated by reference in their entirety).

### LAG-3 antagonist

A LAG-3 antagonist within the meaning of this invention and all of its embodiments is a compound that inhibits the interaction of LAG-3 with its receptor(s).

Preferably, the LAG-3 antagonist within the meaning of this invention and all of its embodiments is an *anti*-LAG-3 antibody.

Preferably, the LAG-3 antagonist, *i.e.* the *anti*-LAG-3 antibody within this invention and all its embodiments is a humanized or fully human *anti*-LAG-3 antibody.

More preferably, the LAG-3 antagonist within this invention and all its embodiments is selected from the group consisting of the following **(C0):**
- BMS-986016 as disclosed in WO 2015/042246; (page 3/4 and Example 1)
- LAG525;
- MK4280;
- an *anti*-LAG-3 antibody (generically and/or specifically) disclosed in WO 2015/138920:
   ∘ any one of the antibodies as defined in table 1 in WO 2015/138920 (page 187)
   ∘ any one of the humanized antibodies as defined in table 1 in WO 2015/138920 (page 187)
   ∘ any one of BAP050-hum01 to BAP050-hum20 as defined in table 1 in WO 2015/138920 (page 187)
   ∘ any one of BAP050-hum01-Ser to BAP050-hum15-Ser and BAP050-hum18-Ser to BAP050-hum20-Ser as defined in table 1 in WO 2015/138920 (page 187)
   ∘ any one of BAP050-Clone-F to BAP050-Clone-J as defined in table 1 in WO 2015/138920 (page 187)
- an anti-LAG-3 antibody LAG3-1, LAG3-2, LAG3-3, LAG3-4 and LAG3-5 as disclosed herein below

Antibodies LAG3-1 to LAG3-5 are antibody molecules defined by the sequences as shown in Table 3, wherein HC denotes the (full length) heavy chain and LC denotes the (full length) light chain:

**Table 3**

| **SEQ ID NO:** | **Sequence name** | **Amino acid sequence** |
|---|---|---|
| 11 | HC of LAG3-1 | QVTLVESGGGVVQPGRSLRLSCAFSGFSLSTSDMGVG WIRQAPGKGLEWVAHIWWDDVKRYNPALKSRFTISRDN SKNTLYLQMNSLRAEDTAVYFCARIEDYGVSYYFDYWG QGTTVTVSSASTKGPSVFPLAPCSRSTSESTAALGCLVK DYFPEPVTVSWNSGALTSGVHTFPAVLQSSGLYSLSSVV TVPSSSLGTKTYTCNVDHKPSNTKVDKRVESKYGPPCP PCPAPEFLGGPSVFLFPPKPKDTLMISRTPEVTCVVVDV SQEDPEVQFNWYVDGVEVHNAKTKPREEQFNSTYRVV SVLTVLHQDWLNGKEYKCKVSNKGLPSSIEKTISKAKGQ PREPQVYTLPPSQEEMTKNQVSLTCLVKGFYPSDIAVEW ESNGQPENNYKTTPPVLDSDGSFFLYSRLTVDKSRWQE GNVFSCSVMHEALHNHYTQKSLSLSLG |
| 12 | LC of LAG3-1 | DIQMTQSPSFLSASVGDRVSITCKASQDVSTAVAWYQQ KPGKAPKLLIYSASYRYTGVPDRFSGSGSGTDFTLTISSL QPEDFATYYCQQHYSIPLTFGQGTKLEIKRTVAAPSVFIF PPSDEQLKSGTASVVCLLNNFYPREAKVQWKVDNALQS GNSQESVTEQDSKDSTYSLSSTLTLSKADYEKHKVYACE VTHQGLSSPVTKSFNRGEC |
| 13 | HC of LAG3-2 | QVTLKESGPTLVKPTQTLTLTCSFSGFSLSTSDMGVGWI RQPPGKALEWLAHIWWDDVKRYNPALKSRLTITKDTSKN QVVLTMTNMDPVDTATYFCARIEDYGVSYYFDYWGQGT TVTVSSASTKGPSVFPLAPCSRSTSESTAALGCLVKDYF PEPVTVSWNSGALTSGVHTFPAVLQSSGLYSLSSVVTVP SSSLGTKTYTCNVDHKPSNTKVDKRVESKYGPPCPPCP APEFLGGPSVFLFPPKPKDTLMISRTPEVTCVVVDVSQE DPEVQFNWYVDGVEVHNAKTKPREEQFNSTYRVVSVLT VLHQDWLNGKEYKCKVSNKGLPSSIEKTISKAKGQPREP QVYTLPPSQEEMTKNQVSLTCLVKGFYPSDIAVEWESN GQPENNYKTTPPVLDSDGSFFLYSRLTVDKSRWQEGNV FSCSVMHEALHNHYTQKSLSLSLG |
| 14 | LC of LAG3-2 | DIQMTQSPSFLSASVGDRVTFTCKASQDVSTAVAWYQQ KPGKAPKLLIYSASYRYTGVPDRFSGSGSGTDFTLTISSL QPEDFATYYCQQHYSIPLTFGQGTKLEIKRTVAAPSVFIF PPSDEQLKSGTASVVCLLNNFYPREAKVQWKVDNALQS GNSQESVTEQDSKDSTYSLSSTLTLSKADYEKHKVYACE VTHQGLSSPVTKSFNRGEC |
| 15 | HC of LAG3-3 | QVTLVESGGGVVQPGRSLSLSCAFSGFSLSTSDMGVG WVRQPPGKGLEWVAHIWWDDVKRYNPALKSRFTISRD NSKNTLYLQMNSLRAEDTATYYCARIEDYGVSYYFDYW GQGTTVTVSSASTKGPSVFPLAPCSRSTSESTAALGCLV KDYFPEPVTVSWNSGALTSGVHTFPAVLQSSGLYSLSSV VTVPSSSLGTKTYTCNVDHKPSNTKVDKRVESKYGPPC PPCPAPEFLGGPSVFLFPPKPKDTLMISRTPEVTCVVVD VSQEDPEVQFNWYVDGVEVHNAKTKPREEQFNSTYRV VSVLTVLHQDWLNGKEYKCKVSNKGLPSSIEKTISKAKG QPREPQVYTLPPSQEEMTKNQVSLTCLVKGFYPSDIAVE WESNGQPENNYKTTPPVLDSDGSFFLYSRLTVDKSRWQ EGNVFSCSVMHEALHNHYTQKSLSLSLG |
| 16 | LC of LAG3-3 | DIQMTQSPSFLSASVGDRVTITCKASQDVSTAVAWYQQ KPGKAPKLLIYSASYRYTGVPDRFSGSGSGTDFTLTISSL QPEDFATYYCQQHYSIPLTFGAGTKLEIKRTVAAPSVFIF PPSDEQLKSGTASVVCLLNNFYPREAKVQWKVDNALQS GNSQESVTEQDSKDSTYSLSSTLTLSKADYEKHKVYACE VTHQGLSSPVTKSFNRGEC |
| 17 | HC of LAG3-4 | QVTLVESGGGVVQPGRSLRLSCAFSGFSLSTSDMGVG WIRQAPGKGLEWVAHIWWDDVKRYNPALKSRFTISRDN SKNTLYLQMNSLRAEDTATYFCARIEDYGVSYYFDYWG QGTTVTVSSASTKGPSVFPLAPCSRSTSESTAALGCLVK DYFPEPVTVSWNSGALTSGVHTFPAVLQSSGLYSLSSVV TVPSSSLGTKTYTCNVDHKPSNTKVDKRVESKYGPPCP PCPAPEFLGGPSVFLFPPKPKDTLMISRTPEVTCVVVDV SQEDPEVQFNWYVDGVEVHNAKTKPREEQFNSTYRVV SVLTVLHQDWLNGKEYKCKVSNKGLPSSIEKTISKAKGQ PREPQVYTLPPSQEEMTKNQVSLTCLVKGFYPSDIAVEW ESNGQPENNYKTTPPVLDSDGSFFLYSRLTVDKSRWQE GNVFSCSVMHEALHNHYTQKSLSLSLG |
| 18 | LC of LAG3-4 | DIVMTQSPSFLSASVGDRVTITCKASQDVSTAVAWYQQK PGKAPKLLIYSASYRYTGVPDRFSGSGSGTDFTLTISSLQ PEDFATYYCQQHYSIPLTFGQGTKLEIKRTVAAPSVFIFP PSDEQLKSGTASVVCLLNNFYPREAKVQWKVDNALQSG NSQESVTEQDSKDSTYSLSSTLTLSKADYEKHKVYACEV THQGLSSPVTKSFNRGEC |
| 19 | HC of LAG3-5 | QVTLKESGPTLVKPTQTLTLTCSFSGFSLSTSDMGVGWI RQPPGKALEWLAHIWWDDVKRYNPALKSRLTITKDTSKN QVVLTMTNMDPVDTATYFCARIVDYGVSYYFDYWGQGT TVTVSSASTKGPSVFPLAPCSRSTSESTAALGCLVKDYF PEPVTVSWNSGALTSGVHTFPAVLQSSGLYSLSSVVTVP SSSLGTKTYTCNVDHKPSNTKVDKRVESKYGPPCPPCP APEFLGGPSVFLFPPKPKDTLMISRTPEVTCVVVDVSQE DPEVQFNWYVDGVEVHNAKTKPREEQFNSTYRVVSVLT VLHQDWLNGKEYKCKVSNKGLPSSIEKTISKAKGQPREP QVYTLPPSQEEMTKNQVSLTCLVKGFYPSDIAVEWESN GQPENNYKTTPPVLDSDGSFFLYSRLTVDKSRWQEGNV FSCSVMHEALHNHYTQKSLSLSLG |
| 20 | LC of LAG3-5 | DIQMTQSPSFLSASVGDRVSITCKASQDVSTAVAWYQQ KPGKAPKLLIYSASYRYTGVPDRFSGSGSGTDFTLTISSL QPEDFAVYYCQQHYSIPLTFGQGTKLEIKRTVAAPSVFIF PPSDEQLKSGTASVVCLLNNFYPREAKVQWKVDNALQS GNSQESVTEQDSKDSTYSLSSTLTLSKADYEKHKVYACE VTHQGLSSPVTKSFNRGEC |

Specifically, the anti-LAG-3 antibody molecule described herein above has:
(LAG3-1) a heavy chain comprising the amino acid sequence of SEQ ID NO:11 and a light chain comprising the amino acid sequence of SEQ ID NO:12; or
(LAG3-2) a heavy chain comprising the amino acid sequence of SEQ ID NO:13 and a light chain comprising the amino acid sequence of SEQ ID NO:14; or
(LAG3-3) a heavy chain comprising the amino acid sequence of SEQ ID NO:15 and a light chain comprising the amino acid sequence of SEQ ID NO:16; or
(LAG3-4) a heavy chain comprising the amino acid sequence of SEQ ID NO:17 and a light chain comprising the amino acid sequence of SEQ ID NO:18; or
(LAG3-5) a heavy chain comprising the amino acid sequence of SEQ ID NO:19 and a light chain comprising the amino acid sequence of SEQ ID NO:20.

All LAG-3 antagonists listed above are known in the art with their respective manufacture, therapeutic use and properties. All patent applications referred to above are incorporated by reference in their entirety.

In one embodiment the LAG-3 antagonist is BMS-986016 **(C1).**

In another embodiment the LAG-3 antagonist is LAG525 **(C2).**

In another embodiment the LAG-3 antagonist is BAP050-Clone-F as defined in table 1 in WO 2015/138920 (page 187) **(C3).**

In another embodiment the LAG-3 antagonist is BAP050-Clone-G as defined in table 1 in WO 2015/138920 (page 187) **(C4).**

In another embodiment the LAG-3 antagonist is BAP050-Clone-H as defined in table 1 in WO 2015/138920 (page 187) **(C5).**

In another embodiment the LAG-3 antagonist is BAP050-Clone-I as defined in table 1 in WO 2015/138920 (page 187) **(C6).**

In another embodiment the LAG-3 antagonist is BAP050-Clone-J as defined in table 1 in WO 2015/138920 (page 187) **(C7).**

In another embodiment the LAG-3 antagonist is LAG3-1 **(C8).**

In another embodiment the LAG-3 antagonist is LAG3-2 **(C9).**

In another embodiment the LAG-3 antagonist is LAG3-3 **(C10).**

In another embodiment the LAG-3 antagonist is LAG3-4 **(C11).**

In another embodiment the LAG-3 antagonist is LAG3-5 **(C12).**

All embodiments **(C1)** to **(C12)** are preferred embodiments of embodiment **(C0)** in respect of the nature of the LAG-3 antagonist.

For *anti*-PD-1 antibodies, *anti*-PD-L1 antibodies and *anti*-LAG-3 antibodies as disclosed/defined herein the term "antibody"/"antibodies" encompasses antibodies, antibody fragments, antibody-like molecules and conjugates with any of the above. Antibodies include, but are not limited to, poly- or monoclonal, chimeric, humanized, human, mono-, bi- or multispecific antibodies. The term "antibody" shall encompass complete immunoglobulins as they are produced by lymphocytes and for example present in blood sera, monoclonal antibodies secreted by hybridoma cell lines, polypeptides produced by recombinant expression in host cells, which have the binding specificity of immunoglobulins or monoclonal antibodies, and molecules which have been derived from such immunoglobulins, monoclonal antibodies, or polypeptides by further processing while retaining their binding specificity. In particular, the term "antibody" includes complete immunoglobulins comprising two heavy chains and two light chains. In another embodiment, the term encompasses a fragment of an immunoglobulin, like Fab fragments. In another embodiment, the term "antibody" encompasses a polypeptide having one or more variable domains derived from an immunobulin, like single chain antibodies (scFv), single domain antibodies, and the like.

To be used in therapy, the respective *anti*-PD1, *anti*-PD-L1 and/or *anti*-LAG-3 antibody molecule is included into pharmaceutical compositions appropriate to facilitate administration to animals or humans.

For pharmaceutical use, the antibody molecules of the invention may be formulated as a pharmaceutical preparation comprising (i) at least one antibody of the invention and (ii) at least one pharmaceutically acceptable carrier, diluent, excipient, adjuvant, and/or stabilizer, and (iii) optionally one or more further pharmacologically active polypeptides and/or compounds. By "pharmaceutically acceptable" is meant that the respective material does not show any biological or otherwise undesirable effects when administered to an individual and does not interact in a deleterious manner with any of the other components of the pharmaceutical composition (such as e.g. the pharmaceutically active ingredient) in which it is contained. Specific examples can be found in standard handbooks, such as e.g. Remington's Pharmaceutical Sciences, 18th Ed., Mack Publishing Company, USA (1990). For example, the antibodies of the invention may be formulated and administered in any manner known *per se* for conventional antibodies and antibody fragments and other pharmaceutically active proteins. Thus, according to a further embodiment, the invention relates to a pharmaceutical composition or preparation that contains at least one antibodys of the invention and at least one pharmaceutically acceptable carrier, diluent, excipient, adjuvant and/or stabilizer, and optionally one or more further pharmacologically active substances.

Pharmaceutical preparations for parenteral administration, such as intravenous, intramuscular, subcutaneous injection or intravenous infusion may for example be sterile solutions, suspensions, dispersions, emulsions, or powders which comprise the active ingredient and which are suitable, optionally after a further dissolution or dilution step, for infusion or injection. Suitable carriers or diluents for such preparations for example include, without limitation, sterile water and pharmaceutically acceptable aqueous buffers and solutions such as physiological phosphate-buffered saline, Ringer's solutions, dextrose solution, and Hank's solution; water oils; glycerol; ethanol; glycols such as propylene glycol, as well as mineral oils, animal oils and vegetable oils, for example peanut oil, soybean oil, as well as suitable mixtures thereof.

Solutions of the antibody molecules of the invention may also contain a preservative to prevent the growth of microorganisms, such as antibacterial and antifungal agents, for example, p-hydroxybenzoates, parabens, chlorobutanol, phenol, sorbic acid, thiomersal, (alkali metal salts of) ethylenediamine tetraacetic acid, and the like. In many cases, it will be preferable to include isotonic agents, for example, sugars, buffers or sodium chloride. Optionally, emulsifiers and/or dispersants may be used. The proper fluidity can be maintained, for example, by the formation of liposomes, by the maintenance of the required particle size in the case of dispersions or by the use of surfactants. Other agents delaying absorption, for example, aluminum monostearate and gelatin, may also be added. The solutions may be filled into injection vials, ampoules, infusion bottles, and the like.

In all cases, the ultimate dosage form must be sterile, fluid and stable under the conditions of manufacture and storage. Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

Usually, aqueous solutions or suspensions will be preferred. Generally, suitable formulations for therapeutic proteins such as the antibodies of the invention are buffered protein solutions, such as solutions including the protein in a suitable concentration (such as from 0.001 to 400 mg/mL, preferably from 0.005 to 200 mg/mL, more preferably 0.01 to 200 mg/mL, more preferably 1.0 - 100 mg/mL, such as 1.0 mg/mL (i.v. administration) or 100 mg/mL (s.c. administration) and an aqueous buffer such as:
- phosphate buffered saline, pH 7.4,
- other phosphate buffers, pH 6.2 to 8.2,
- acetate buffers, pH 3.2 to 7.5, preferably pH 4.8 to 5.5
- histidine buffers, pH 5.5 to 7.0,
- succinate buffers, pH 3.2 to 6.6, and
- citrate buffers, pH 2.1 to 6.2,
and, optionally, salts (*e.g*. NaCl) and/or sugars (such as *e.g.* sucrose and trehalose) and/or other polyalcohols (such as *e.g*. mannitol and glycerol) for providing isotonicity of the solution.

Preferred buffered protein solutions are solutions including about 0.05 mg/mL of the antibody of the invention dissolved in 25 mM phosphate buffer, pH 6.5, adjusted to isotonicity by adding 220 mM trehalose. In addition, other agents such as a detergent, *e.g*. 0.02 % Tween-20 or Tween-80, may be included in such solutions. Formulations for subcutaneous application may include significantly higher concentrations of the antibody of the invention, such as up to 100 mg/mL or even above 100 mg/mL. However, it will be clear to the person skilled in the art that the ingredients and the amounts thereof as given above do only represent one, preferred option. Alternatives and variations thereof will be immediately apparent to the skilled person, or can easily be conceived starting from the above disclosure.

The antibody may be administered to the patient at a dose between 1 mg/kg to 20 mg/kg, by one or more separate administrations, or by continuous infusion, *e.g*. infusion over 1 hour. A typical treatment schedule usually involves administration of the antibody once every week to once every three weeks.

### Combination therapy

Within this invention it is to be understood that the combinations, compositions, kits, methods, uses or compounds for use according to this invention may envisage the simultaneous, concurrent, sequential, successive, alternate or separate administration of the active ingredients or components. It will be appreciated that the MDM2 inhibitor, the PD-1 antagonist and the LAG-3 antagonist can be administered formulated either dependently or independently, such as e.g. the MDM2 inhibitor, the PD-1 antagonist and the LAG-3 antagonist may be administered either as part of the same pharmaceutical composition/dosage form or, preferably, in separate pharmaceutical compositions/dosage forms or two may be provided as part of the same pharmaceutical composition/dosage form whereas the third is provided in a separate pharmaceutical compositions/dosage form.

In this context, "combination" or "combined" within the meaning of this invention includes, without being limited, a product that results from the mixing or combining of more than one active ingredient and includes both fixed and non-fixed (*e.g*. free) combinations (including kits) and uses, such as *e.g*. the simultaneous, concurrent, sequential, successive, alternate or separate use of the components or ingredients. The term "fixed combination" means that the active ingredients are both administered to a patient simultaneously in the form of a single entity or dosage. The term "non-fixed combination" means that the active ingredients are both administered to a patient as separate entities either simultaneously, concurrently or sequentially with no specific time limits, wherein such administration provides therapeutically effective levels of the two compounds in the body of the patient. The latter also applies to cocktail therapy, *e.g.* the administration of three or more active ingredients.

The administration of the MDM2 inhibitor, PD-1 antagonist and LAG-3 antagonist may take place by co-administering the active components or ingredients, such as *e.g*. by administering them simultaneously or concurrently in one single or in two or three separate formulations or dosage forms. Alternatively, the administration of the MDM2 inhibitor, the PD-1 antagonist and the LAG-3 antagonist may take place by administering the active components or ingredients sequentially or in alternation, such as *e.g*. in two or three separate formulations or dosage forms.

For example, simultaneous administration includes administration at substantially the same time. This form of administration may also be referred to as "concomitant" administration. Concurrent administration includes administering the active agents within the same general time period, for example on the same day(s) but not necessarily at the same time. Alternate administration includes administration of one agent during a time period, for example over the course of a few days or a week, followed by administration of the other agents during a subsequent period of time, for example over the course of a few days or a week, and then repeating the pattern for one or more cycles. Sequential or successive administration includes administration of one agent during a first time period (for example over the course of a few days or a week) using one or more doses, followed by administration of the other agents during a second and third time period (for example over the course of a few days or a week) using one or more doses. An overlapping schedule may also be employed, which includes administration of the active agents on different days over the treatment period, not necessarily according to a regular sequence. Variations on these general guidelines may also be employed, *e.g*. according to the agents used and the condition of the subject.

The aforementioned applies accordingly if the combination setting is not a triple but a higher multiple combination approach.

The elements of the combinations of this invention may be administered (whether dependently or independently) by methods customary to the skilled person, *e.g*. by oral, enterical, parenteral (*e.g.*, intramuscular, intraperitoneal, intravenous, transdermal or subcutaneous injection, or implant), nasal, vaginal, rectal, or topical routes of administration and may be formulated, alone or together, in suitable dosage unit formulations containing conventional non-toxic pharmaceutically acceptable carriers, excipients and/or vehicles appropriate for each route of administration.

Accordingly, in one aspect of the invention the invention provides a method of treating and/or preventing an oncological or hyperproliferative disease, in particular cancer, as described herein, comprising administering to a patient in need thereof a therapeutically effective amount of an MDM2 inhibitor, a therapeutically effective amount of a PD-1 antagonist and a therapeutically effective amount of a LAG-3 antagonist and, optionally, one or more additional therapeutic agent(s), each as described herein, wherein the MDM2 inhibitor is administered simultaneously, concurrently, sequentially, successively, alternately or separately with the PD-1 antagonist, the LAG-3 antagonist and the optional one or more additional therapeutic agent(s) if present.

In another aspect the invention provides an MDM2 inhibitor as described herein for use in a method of treating and/or preventing an oncological or hyperproliferative disease, in particular cancer, as described herein, said method comprising administering the MDM2 inhibitor in combination with a PD-1 antagonist and a LAG-3 antagonist and, optionally, one or more additional therapeutic agent(s), each as described herein, wherein the MDM2 inhibitor is administered simultaneously, concurrently, sequentially, successively, alternately or separately with the PD-1 antagonist, the LAG-3 antagonist and the optional one or more additional therapeutic agent(s) if present.

In another aspect the invention provides a PD-1 antagonist as described herein for use in a method of treating and/or preventing an oncological or hyperproliferative disease, in particular cancer, as described herein, said method comprising administering the PD-1 antagonist in combination with an MDM2 inhibitor, a LAG-3 antagonist and, optionally, one or more additional therapeutic agent(s), each as described herein, wherein the PD-1 antagonist is administered simultaneously, concurrently, sequentially, successively, alternately or separately with the MDM2 inhibitor, LAG-3 antagonist and the optional one or more additional therapeutic agent(s) if present.

In another aspect the invention provides a LAG-3 antagonist as described herein for use in a method of treating and/or preventing an oncological or hyperproliferative disease, in particular cancer, as described herein, said method comprising administering the LAG-3 antagonist in combination with an MDM2 inhibitor, a PD-1 antagonist and, optionally, one or more additional therapeutic agent(s), each as described herein, wherein the LAG-3 antagonist is administered simultaneously, concurrently, sequentially, successively, alternately or separately with the MDM2 inhibitor, PD-1 antagonist and the optional one or more additional therapeutic agent(s) if present.

In another aspect the invention provides the use of an MDM2 inhibitor as described herein for preparing a pharmaceutical composition for use in a method of treating and/or preventing an oncological or hyperproliferative disease, in particular cancer, as described herein, wherein the MDM2 inhibitor is to be used in combination with a PD-1 antagonist, a LAG-3 antagonist and, optionally, one or more additional therapeutic agent(s), each as described herein, and wherein the MDM2 inhibitor is to be administered simultaneously, concurrently, sequentially, successively, alternately or separately with the PD-1 antagonist, the LAG-3 antagonist and the optional one or more additional therapeutic agent(s) if present.

In another aspect the invention provides the use of a PD-1 antagonist as described herein for preparing a pharmaceutical composition for use in a method of treating and/or preventing an oncological or hyperproliferative disease, in particular cancer, as described herein, wherein the PD-1 antagonist is to be used in combination with an MDM2 inhibitor, a LAG-3 antagonist and, optionally, one or more additional therapeutic agent(s), each as described herein, and wherein the PD-1 antagonist is to be administered simultaneously, concurrently, sequentially, successively, alternately or separately with the MDM2 inhibitor, LAG-3 antagonist and the optional one or more additional therapeutic agent(s) if present.

In another aspect the invention provides the use of a LAG-3 antagonist as described herein for preparing a pharmaceutical composition for use in a method of treating and/or preventing an oncological or hyperproliferative disease, in particular cancer, as described herein, wherein the LAG-3 antagonist is to be used in combination with an MDM2 inhibitor, a PD-1 antagonist and, optionally, one or more additional therapeutic agent(s), each as described herein, and wherein the LAG-3 antagonist is to be administered simultaneously, concurrently, sequentially, successively, alternately or separately with the MDM2 inhibitor, PD-1 antagonist and the optional one or more additional therapeutic agent(s) if present.

In another aspect the invention provides a kit comprising
- a first pharmaceutical composition or dosage form comprising an MDM2 inhibitor as described herein, and, optionally, one or more pharmaceutically acceptable carriers, excipients and/or vehicles,
- a second pharmaceutical composition or dosage form comprising a PD-1 antagonist as described herein, and, optionally, one or more pharmaceutically acceptable carriers, excipients and/or vehicles,
- a third pharmaceutical composition or dosage form comprising a PD-1 antagonist as described herein, and, optionally, one or more pharmaceutically acceptable carriers, excipients and/or vehicles, and, optionally
- one or more additional pharmaceutical composition(s) or dosage form(s) each comprising one additional therapeutic agent as described herein, and, optionally, one or more pharmaceutically acceptable carriers, excipients and/or vehicles,
for use in a method of treating and or/preventing an oncological or hyperproliferative disease, in particular cancer, as described herein, wherein the first pharmaceutical composition is to be administered simultaneously, concurrently, sequentially, successively, alternately or separately with the second and third pharmaceutical composition or dosage form and the optional one or more additional pharmaceutical composition(s) or dosage form(s) if present.

In a further embodiment of the invention the components (*i.e*. the combination partners) of the combinations, kits, uses, methods and compounds for use according to the invention (including all embodiments) are administered simultaneously.

In a further embodiment of the invention the components *(i.e.* the combination partners) of the combinations, kits, uses, methods and compounds for use according to the invention (including all embodiments) are administered concurrently.

In a further embodiment of the invention the components *(i.e.* the combination partners) of the combinations, kits, uses, methods and compounds for use according to the invention (including all embodiments) are administered sequentially.

In a further embodiment of the invention the components *(i.e.* the combination partners) of the combinations, kits, uses, methods and compounds for use according to the invention (including all embodiments) are administered successively.

In a further embodiment of the invention the components *(i.e.* the combination partners) of the combinations, kits, uses, methods and compounds for use according to the invention (including all embodiments) are administered alternately.

In a further embodiment of the invention the components (*i.e*. the combination partners) of the combinations, kits, uses, methods and compounds for use according to the invention (including all embodiments) are administered separately.

In a preferred embodiment the MDM2 inhibitor as described herein is to be administered orally.

In another preferred embodiment the PD-1 antagonist as described herein is to be administered intravenously.

In another preferred embodiment the LAG-3 antagonist as described herein is to be administered intravenously.

The "therapeutically effective amount" of the active compound(s) to be administered is the minimum amount necessary to prevent, ameliorate, or treat a disease or disorder.

The combinations of this invention may be administered at therapeutically effective single or divided daily doses. The active components of the combination may be administered in such doses which are therapeutically effective in monotherapy, or in such doses which are lower than the doses used in monotherapy, but when combined result in a desired (joint) therapeutically effective amount.

The permutation of embodiments **(A0)** to **(A47)** (in respect of the MDM2 inhibitor) with embodiments **(B0)** to **(B15)** (in respect of the PD-1 antagonist) and embodiments **(C0)** to **(C12)** results in specific triple combinations or groups of triple combinations D0 to D9984 (D0 = A0B0C0, D1 = A0B0C1, D2 = A0B0C2 *etc*.) which shall all be deemed to be specifically disclosed and to be embodiments of the invention and of all of its combinations, compositions, kits, methods, uses and compounds for use.

### Additional therapeutic agent(s)

The combinations, compositions, kits, uses, methods and compounds for use according to the present invention (including all embodiments) including an MDM2 inhibitor, a PD-1 antagonist and a LAG-3 antagonist, each as described herein, may optionally include one or more additional therapeutic agent(s).

This/these additonal therapeutic agent(s) may (each) be selected from the following (without being limited thereto):
- an immunotherapeutic agent, such as modulators of the following checkpoint inhibitors: TIM3, PD-L1, PD-L2, CTLA-4, VISTA, BTLA, TIGIT, CD160, LAIR1, 2B4, CEACAM;
- a cancer vaccine;
- a DNA damaging agent;
- an inhibitor of angiogenesis;
- an inhibitor of signal transduction pathways;
- an inhibitor of mitotic checkpoints; and
hormones, hormone analogues and antihormones (*e.g*. tamoxifen, toremifene, raloxifene, fulvestrant, megestrol acetate, flutamide, nilutamide, bicalutamide, aminoglutethimide, cyproterone acetate, finasteride, buserelin acetate, fludrocortisone, fluoxymesterone, medroxyprogesterone, octreotide), aromatase inhibitors (*e.g*. anastrozole, letrozole, liarozole, vorozole, exemestane, atamestane), LHRH agonists and antagonists (*e.g*. goserelin acetate, luprolide), inhibitors of growth factors (growth factors such as for example "platelet derived growth factor (PDGF)", "fibroblast growth factor (FGF)", "vascular endothelial growth factor (VEGF)", "epidermal growth factor (EGF)", "insuline-like growth factors (IGF)", "human epidermal growth factor (HER, *e.g*. HER2, HER3, HER4)" and "hepatocyte growth factor (HGF)"), inhibitors are for example "growth factor" antibodies, "growth factor receptor" antibodies and tyrosine kinase inhibitors, such as for example cetuximab, gefitinib, imatinib, lapatinib, bosutinib and trastuzumab); antimetabolites (*e.g*. antifolates such as methotrexate, raltitrexed, pyrimidine analogues such as 5-fluorouracil (5-FU), capecitabine and gemcitabine, purine and adenosine analogues such as mercaptopurine, thioguanine, cladribine and pentostatin, cytarabine (ara C), fludarabine); antitumour antibiotics (*e.g*. anthracyclins such as doxorubicin, doxil (pegylated liposomal doxorubicin hydrochloride, myocet (non-pegylated liposomal doxorubicin), daunorubicin, epirubicin and idarubicin, mitomycin-C, bleomycin, dactinomycin, plicamycin, streptozocin); platinum derivatives (*e.g*. cisplatin, oxaliplatin, carboplatin); alkylation agents (*e.g*. estramustin, meclorethamine, melphalan, chlorambucil, busulphan, dacarbazin, cyclophosphamide, ifosfamide, temozolomide, nitrosoureas such as for example carmustin and lomustin, thiotepa); antimitotic agents (*e.g*. Vinca alkaloids such as for example vinblastine, vindesin, vinorelbin and vincristine; and taxanes such as paclitaxel, docetaxel); angiogenesis inhibitors (*e.g.* tasquinimod), tubuline inhibitors; DNA synthesis inhibitors (*e.g*. sapacitabine), PARP inhibitors, topoisomerase inhibitors (*e.g*. epipodophyllotoxins such as for example etoposide and etopophos, teniposide, amsacrin, topotecan, irinotecan, mitoxantrone), serine/threonine kinase inhibitors (*e.g*. PDK 1 inhibitors,Raf inhibitors, A-Raf inhibitros, B-Raf inhibitors, C-Raf inhibitors, mTOR inhibitors, mTORC1/2 inhibitors, PI3K inhibitors, PI3Kα inhibitors, dual mTOR/PI3K inhibitors, STK 33 inhibitors, AKT inhibitors, PLK 1 inhibitors, inhibitors of CDKs, *e.g.* inhibitors of CDK4/6, Aurora kinase inhibitors), tyrosine kinase inhibitors (*e.g*. PTK2/FAK inhibitors), protein protein interaction inhibitors (*e.g.* IAP activator, Mcl-1, MDM2/MDMX), MEK inhibitors (*e.g*. pimasertib), ERK inhibitors, FLT3 inhibitors (*e.g*. quizartinib), BRD4 inhibitors, IGF-1R inhibitors, TRAILR2 agonists, Bcl-xL inhibitors, Bcl-2 inhibitors (*e.g*. venetoclax), Bcl-2/Bcl-xL inhibitors, ErbB receptor inhibitors, BCR-ABL inhibitors, ABL inhibitors, Src inhibitors, rapamycin analogs (*e.g*. everolimus, temsirolimus, ridaforolimus, sirolimus), androgen synthesis inhibitors (*e.g*. abiraterone, TAK-700), androgen receptor inhibitors (*e.g*. enzalutamide, ARN-509), immunotherapy (*e.g*. sipuleucel-T), DNMT inhibitors (*e.g*. SGI 110, temozolomide, vosaroxin), HDAC inhibitors (e.g. vorinostat, entinostat, pracinostat, panobinostat), ANG1/2 inhibitors (*e.g*. trebananib), CYP17 inhibitors (*e.g*. galeterone), radiopharmaceuticals (*e.g*. radium-223, alpharadin), immunotherapeutic agents (*e.g*. poxvirus-based vaccine, ipilimumab, immune checkpoint inhibitors) and various chemotherapeutic agents such as amifostin, anagrelid, clodronat, filgrastin, interferon, interferon alpha, leucovorin, rituximab, procarbazine, levamisole, mesna, mitotane, pamidronate and porfimer;
2-chlorodesoxyadenosine, 2-fluorodesoxycytidine, 2-methoxyoestradiol, 2C4, 3-alethine, 131-I-TM-601, 3CPA, 7-ethyl-10-hydroxycamptothecin, 16-aza-epothilone B, abemaciclib, ABT-199, ABT-263/navitoclax, ABT-737, A 105972, A 204197, aldesleukin, alisertib/MLN8237, alitretinoin, allovectin-7, altretamine, alvocidib, amonafide, anthrapyrazole, AG-2037, AP-5280, apaziquone, apomine, aranose, arglabin, arzoxifene, atamestane, atrasentan, auristatin PE, AVLB, AZ10992, ABX-EGF, AMG-479 (ganitumab), AMG-232, AMG-511, AMG 2520765, AMG 2112819, ARRY 162, ARRY 438162, ARRY-300, ARRY-142886/AZD-6244 (selumetinib), ARRY-704/ AZD-8330, ATSP-7041, AR-12, AR-42, AS-703988, AXL-1717, AZD-1480, AZD-4547, AZD-8055, AZD-5363, AZD-6244, AZD-7762, ARQ-736, ARQ 680, AS-703026 (primasertib), avastin, AZD-2014, azacitidine (5-aza), azaepothilone B, azonafide, barasertib/AZD1152, BAY-43-9006, BAY 80-6946, BBR-3464, BBR-3576, bevacizumab, BEZ-235/dactolisib, biricodar dicitrate, birinapant, BCX-1777, BKM-120/buparlisib, bleocin, BLP-25, BMS-184476, BMS-247550, BMS-188797, BMS-275291, BMS-663513, BMS-754807, BNP-1350, BNP-7787, BIBW 2992/afatinib, BIBF 1120/nintedanib, BI 836845, BI 2536, BI 6727/volasertib, BI 836845, BI 847325, BI 853520, BIIB-022, bleomycinic acid, bleomycin A, bleomycin B, brivanib, bryostatin-1, bortezomib, brostallicin, busulphan, BYL-719/alpelisib, CA-4 prodrug, CA-4, cabazitaxel, cabozantinib, CapCell, calcitriol, canertinib, canfosfamide, capecitabine, carboxyphthalatoplatin, CCI-779, CC-115, CC-223, CEP-701, CEP-751, CBT-1 cefixime, ceflatonin, ceftriaxone, celecoxib, celmoleukin, cemadotin, CGM-097, CH4987655/RO-4987655, chlorotrianisene, cilengitide, ciclosporin, CD20 antibodies, CDA-II, CDC-394, CKD-602, CKI-27, clofarabine, colchicin, combretastatin A4, COT inhibitors, CHS-828, CH-5132799, CLL-Thera, CMT-3 cryptophycin 52, CPI-613, CTP-37, CTLA-4 monoclonal antibodies (e.g. ipilimumab), CP-461, crizotinib, CV-247, cyanomorpholinodoxorubicin, cytarabine, D 24851, dasatinib, decitabine, deoxorubicin, deoxyrubicin, deoxycoformycin, depsipeptide, desoxyepothilone B, dexamethasone, dexrazoxanet, diethylstilbestrol, diflomotecan, didox, DMDC, dolastatin 10, doranidazole, DS-7423, DS-3032, E7010, E-6201, edatrexat, edotreotide, efaproxiral, eflornithine, EGFR inhibitors, EKB-569, EKB-509, enzastaurin, elesclomol, elsamitrucin, epothilone B, epratuzumab, EPZ-004777, ER-86526, erlotinib, ET-18-OCH3, ethynylcytidine, ethynyloestradiol, exatecan, exatecan mesylate, exemestane, exisulind, fenretinide, figitumumab, floxuridine, folic acid, FOLFOX, FOLFOX4, FOLFIRI, formestane, fostamatinib, fotemustine, galarubicin, gallium maltolate, ganetespib, gefinitib, gemtuzumab, gemtuzumab ozogamicin, gimatecan, glufosfamide, GCS-IOO, GDC-0623, GDC-0941 (pictrelisib), GDC-0980, GDC-0032, GDC-0068, GDC-0349, GDC-0879, G17DT immunogen, GMK, GMX-1778, GPX-100, gp100-peptide vaccines, GSK-5126766, GSK-690693, GSK-1120212 (trametinib), GSK-1995010, GSK-2118436 (dabrafenib), GSK-2126458, GSK-2132231A, GSK-2334470, GSK-2110183, GSK-2141795, GSK-2636771, GSK-525762A/I-BET-762, GW2016, granisetron, herceptine, hexamethylmelamine, histamine, homoharringtonine, hyaluronic acid, hydroxyurea, hydroxyprogesterone caproate,HDM-201, ibandronate, ibritumomab, ibrutinib/PCI-32765, idasanutlin, idatrexate, idelalisib/CAL-101, idenestrol, IDN-5109, IGF-1R inhibitors, IMC-1C11, IMC-A12 (cixutumumab), immunol, indisulam, interferon alpha-2a, interferon alpha-2b, pegylated interferon alpha-2b, interleukin-2, INK-1117, INK-128, INSM-18, ionafarnib, iproplatin, irofulven, isohomohalichondrin-B, isoflavone, isotretinoin, ixabepilone, JRX-2, JSF-154, JQ-1, J-107088, conjugated oestrogens, kahalid F, ketoconazole, KW-2170, KW-2450, KU-55933, LCL-161, lobaplatin, leflunomide, lenalidomide, lenograstim, leuprolide, leuporelin, lexidronam, LGD-1550, linezolid, lovastatin, lutetium texaphyrin, lometrexol, lonidamine, losoxantrone, LU 223651, lurbinectedin, lurtotecan, LY-S6AKT1, LY-2780301, LY-2109761/galunisertib, mafosfamide, marimastat, masoprocol, mechloroethamine, MEK inhibitors, MEK-162, methyltestosteron, methylprednisolone, MEDI-573, MEN-10755, MDX-H210, MDX-447, MDX-1379, MGV, midostaurin, minodronic acid, mitomycin, mivobulin, MK-2206, MK-0646 (dalotuzumab), MLN518, MLN-0128, MLN-2480, motexafin gadolinium, MS-209, MS-275, MX6, neridronate, neratinib, Nexavar, neovastat, nilotinib, nimesulide, nitroglycerin, nolatrexed, norelin, N-acetylcysteine, NU-7441 06-benzylguanine, oblimersen, omeprazole, olaparib, oncophage, oncoVEX^{GM-CSF}, ormiplatin, ortataxel, OX44 antibodies, OSI-027, OSI-906 (linsitinib), 4-1 BB antibodies, oxantrazole, oestrogen, onapristone, palbociclib/PD-0332991, panitumumab, panobinostat, patupilone, pazopanib, pegfilgrastim, PCK-3145, pegfilgrastim, PBI-1402, PBI-05204, PD0325901, PD-1 and PD-L1 antibodies (*e.g*. pembrolizumab, nivolumab, pidilizumab, MEDI-4736/durvalumab, RG-7446/atezolizumab), PD-616, PEG-paclitaxel, albumin-stabilized paclitaxel, PEP-005, PF-05197281, PF-05212384, PF-04691502, PF-3758309, PHA-665752, PHT-427, P-04, PKC412, P54, PI-88, pelitinib, pemetrexed, pentrix, perifosine, perillylalcohol, pertuzumab, pevonedistat, PI3K inhibitors, PI3K/mTOR inhibitors, PG-TXL, PG2, PLX-4032/RO-5185426 (vemurafenib), PLX-3603/RO-5212054, PT-100, PWT-33597, PX-866, picoplatin, pivaloyloxymethylbutyrate, pixantrone, phenoxodiol O, PKI166, plevitrexed, plicamycin, polyprenic acid, ponatinib, porfiromycin, posaconazole, prednisone, prednisolone, PRT-062607, quinamed, quinupristin, quizartinib/AC220, R115777, RAF-265, ramosetron, ranpirnase, RDEA-119/BAY 869766, RDEA-436, rebeccamycin analogues, receptor tyrosine kinase (RTK) inhibitors, revimid, RG-7167, RG-7112, RG-7304, RG-7421, RG-7321, RG-7356, RG 7440, RG-7775, rhizoxin, rhu-MAb, ribociclib, rigosertib rinfabate, risedronate, rituximab, robatumumab, rofecoxib, romidepsin, RO-4929097, RO-31-7453, RO-5126766, RO-5068760, RPR 109881A, rubidazone, rubitecan, R-flurbiprofen, RX-0201, ruxolitinib, S-9788, sabarubicin, SAHA, sapacitabine, SAR-405838, sargramostim, satraplatin, SB-408075, SB-431542, Se-015/Ve-015, SU5416, SU6668, SDX-101, selinexor, semustin, seocalcitol, SM-11355, SN-38, SN-4071, SR-27897, SR-31747, SR-13668, SRL-172, sorafenib, spiroplatin, squalamine, STF-31, suberanilohydroxamic acid, sutent, T 900607, T 138067, TAE-684, TAK-733, TAS-103, tacedinaline, talaporfin, tanespimycin, Tarceva, tariquitar, tasisulam, taxotere, taxoprexin, tazarotene, tegafur, temozolamide, tesmilifene, testosterone, testosterone propionate, tesmilifene, tetraplatin, tetrodotoxin, tezacitabine, thalidomide, theralux, therarubicin, thymalfasin, thymectacin, tiazofurin, tipifarnib, tirapazamine, tocladesine, tomudex, toremofin, tosedostat. trabectedin, TransMID-107, transretinic acid, traszutumab, tremelimumab, tretinoin, triacetyluridine, triapine, triciribine, trimetrexate, TLK-286TXD 258, tykerb/tyverb, urocidin, valproic acid, valrubicin, vandetanib, vatalanib, vincristine, vinflunine, virulizin, vismodegib, vosaroxin, WX-UK1, WX-554, vectibix, XAV-939, xeloda, XELOX, XL-147, XL-228, XL-281, XL-518/R-7420/GDC-0973, XL-765, YM-511, YM-598, ZD-4190, ZD-6474, ZD-4054, ZD-0473, ZD-6126, ZD-9331, ZDI839, ZSTK-474, zoledronat and zosuquidar.

### Oncological or hyperproliferative diseases/cancers

The combinations, compositions, kits, uses, methods and compounds for use according to the present invention (including all embodiments) are useful for the treatment and/or prevention of oncological and hyperproliferative disorders.

In certain embodiments the combinations, compositions, kits, uses, methods and compounds for use according to the present invention (including all embodiments) are useful for the treatment of oncological and hyperproliferative disorders.

In certain embodiments, the hyperproliferative disorder is cancer.

Cancers are classified in two ways: by the type of tissue in which the cancer originates (histological type) and by primary site, or the location in the body, where the cancer first developed. The most common sites in which cancer develops include the skin, lung, breast, prostate, colon and rectum, cervix and uterus as well as the hematological compartment.

The combinations, compositions, kits, uses, methods and compounds for use according to the invention (including all embodiments) may be useful in the treatment of a variety of oncological and hyperproliferative disorders, in particular cancers, including, for example, but not limited to the following:
- Cancers/tumors/carcinomas of the head and neck: e.g. tumors/carcinomas/cancers of the nasal cavity, paranasal sinuses, nasopharynx, oral cavity (including lip, gum, alveolar ridge, retromolar trigone, floor of mouth, tongue, hard palate, buccal mucosa), oropharynx (including base of tongue, tonsil, tonsillar pilar, soft palate, tonsillar fossa, pharyngeal wall), middle ear, larynx (including supraglottis, glottis, subglottis, vocal cords), hypopharynx, salivary glands (including minor salivary glands);
- cancers/tumors/carcinomas of the lung: *e.g.* non-small cell lung cancer (NSCLC) (squamous cell carcinoma, spindle cell carcinoma, adenocarcinoma, large cell carcinoma, clear cell carcinoma, bronchioalveolar), small cell lung cancer (SCLC) (oat cell cancer, intermediate cell cancer, combined oat cell cancer);
- neoplasms of the mediastinum: *e.g.* neurogenic tumors (including neurofibroma, neurilemoma, malignant schwannoma, neurosarcoma, ganglioneuroblastoma, ganglioneuroma, neuroblastoma, pheochromocytoma, paraganglioma), germ cell tumors (including seminoma, teratoma, non-seminoma), thymic tumors (including thymoma, thymolipoma, thymic carcinoma, thymic carcinoid), mesenchymal tumors (including fibroma, fibrosarcoma, lipoma, liposarcoma, myxoma, mesothelioma, leiomyoma, leiomyosarcoma, rhabdomyosarcoma, xanthogranuloma, mesenchymoma, hemangioma, hemangioendothelioma, hemangiopericytoma, lymphangioma, lymphangiopericytoma, lymphangiomyoma);
- cancers/tumors/carcinomas of the gastrointestinal (GI) tract: *e.g.* tumors/carcinomas/ cancers of the esophagus, stomach (gastric cancer), pancreas, liver and biliary tree (including hepatocellular carcinoma (HCC), e.g. childhood HCC, fibrolamellar HCC, combined HCC, spindle cell HCC, clear cell HCC, giant cell HCC, carcinosarcoma HCC, sclerosing HCC; hepatoblastoma; cholangiocarcinoma; cholangiocellular carcinoma; hepatic cystadenocarcinoma; angiosarcoma, hemangioendothelioma, leiomyosarcoma, malignant schwannoma, fibrosarcoma, Klatskin tumor), gall bladder, extrahepatic bile ducts, small intestine (including duodenum, jejunum, ileum), large intestine (including cecum, colon, rectum, anus; colorectal cancer, gastrointestinal stroma tumor (GIST)), genitourinary system (including kidney, *e.g.* renal pelvis, renal cell carcinoma (RCC), nephroblastoma (Wilms' tumor), hypernephroma, Grawitz tumor; ureter; urinary bladder, *e.g.* urachal cancer, urothelial cancer; urethra, *e.g.* distal, bulbomembranous, prostatic; prostate (androgen dependent, androgen independent, castration resistant, hormone independent, hormone refractory), penis);
- cancers/tumors/carcinomas of the testis: *e.g.* seminomas, non-seminomas,
- Gynecologic cancers/tumors/carcinomas: *e.g.* tumors/carcinomas/cancers of the ovary, fallopian tube, peritoneum, cervix, vulva, vagina, uterine body (including endometrium, fundus);
- cancers/tumors/carcinomas of the breast: *e.g.* mammary carcinoma (infiltrating ductal, colloid, lobular invasive, tubular, adenocystic, papillary, medullary, mucinous), hormone receptor positive breast cancer (estrogen receptor positive breast cancer, progesterone receptor positive breast cancer), Her2 positive breast cancer, triple negative breast cancer, Paget's disease of the breast;
- cancers/tumors/carcinomas of the endocrine system: e.g. tumors/carcinomas/cancers of the endocrine glands, thyroid gland (thyroid carcinomas/tumors; papillary, follicular, anaplastic, medullary), parathyroid gland (parathyroid carcinoma/tumor), adrenal cortex (adrenal cortical carcinoma/tumors), pituitary gland (including prolactinoma, craniopharyngioma), thymus, adrenal glands, pineal gland, carotid body, islet cell tumors, paraganglion, pancreatic endocrine tumors (PET; non-functional PET, PPoma, gastrinoma, insulinoma, VIPoma, glucagonoma, somatostatinoma, GRFoma, ACTHoma), carcinoid tumors;
- sarcomas of the soft tissues: *e.g.* fibrosarcoma, fibrous histiocytoma, liposarcoma, leiomyosarcoma, rhabdomyosarcoma, angiosarcoma, lymphangiosarcoma, Kaposi's sarcoma, glomus tumor, hemangiopericytoma, synovial sarcoma, giant cell tumor of tendon sheath, solitary fibrous tumor of pleura and peritoneum, diffuse mesothelioma, malignant peripheral nerve sheath tumor (MPNST), granular cell tumor, clear cell sarcoma, melanocytic schwannoma, plexosarcoma, neuroblastoma, ganglioneuroblastoma, neuroepithelioma, extraskeletal Ewing's sarcoma, paraganglioma, extraskeletal chondrosarcoma, extraskeletal osteosarcoma, mesenchymoma, alveolar soft part sarcoma, epithelioid sarcoma, extrarenal rhabdoid tumor, desmoplastic small cell tumor;
- sarcomas of the bone: *e.g.* myeloma, reticulum cell sarcoma, chondrosarcoma (including central, peripheral, clear cell, mesenchymal chondrosarcoma), osteosarcoma (including parosteal, periosteal, high-grade surface, small cell, radiation-induced osteosarcoma, Paget's sarcoma), Ewing's tumor, malignant giant cell tumor, adamantinoma, (fibrous) histiocytoma, fibrosarcoma, chordoma, small round cell sarcoma, hemangioendothelioma, hemangiopericytoma, osteochondroma, osteoid osteoma, osteoblastoma, eosinophilic granuloma, chondroblastoma;
- mesothelioma: *e.g.* pleural mesothelioma, peritoneal mesothelioma;
- cancers of the skin: e.g. basal cell carcinoma, squamous cell carcinoma, Merkel's cell carcinoma, melanoma (including cutaneous, superficial spreading, lentigo maligna, acral lentiginous, nodular, intraocular melanoma), actinic keratosis, eyelid cancer;
- neoplasms of the central nervous system and brain: e.g. astrocytoma (cerebral, cerebellar, diffuse, fibrillary, anaplastic, pilocytic, protoplasmic, gemistocytary), glioblastoma, gliomas, oligodendrogliomas, oligoastrocytomas, ependymomas, ependymoblastomas, choroid plexus tumors, medulloblastomas, meningiomas, schwannomas, hemangioblastomas, hemangiomas, hemangiopericytomas, neuromas, ganglioneuromas, neuroblastomas, retinoblastomas, neurinomas (e.g. acoustic), spinal axis tumors;
- lymphomas and leukemias: e.g. B-cell non-Hodgkin lymphomas (NHL) (including small lymphocytic lymphoma (SLL), lymphoplasmacytoid lymphoma (LPL), mantle cell lymphoma (MCL), follicular lymphoma (FL), diffuse large cell lymphoma (DLCL), Burkitt's lymphoma (BL)), T-cell non-Hodgkin lymphomas (including anaplastic large cell lymphoma (ALCL), adult T-cell leukemia/lymphoma (ATLL), cutaneous T-cell lymphoma (CTCL), peripheral T-cell lymphoma (PTCL)), lymphoblastic T-cell lymphoma (T-LBL), adult T-cell lymphoma, lymphoblastic B-cell lymphoma (B-LBL), immunocytoma, chronic B-cell lymphocytic leukemia (B-CLL), chronic T-cell lymphocytic leukemia (T-CLL) B-cell small lymphocytic lymphoma (B-SLL), cutaneous T-cell lymphoma (CTLC), primary central nervous system lymphoma (PCNSL), immunoblastoma, Hodgkin's disease (HD) (including nodular lymphocyte predominance HD (NLPHD), nodular sclerosis HD (NSHD), mixed-cellularity HD (MCHD), lymphocyte-rich classic HD, lymphocyte-depleted HD (LDHD)), large granular lymphocyte leukemia (LGL), chronic myelogenous leukemia (CML), acute myelogenous/myeloid leukemia (AML), acute lymphatic/lymphoblastic leukemia (ALL), acute promyelocytic leukemia (APL), chronic lymphocytic/lymphatic leukemia (CLL), prolymphocytic leukemia (PLL), hairy cell leukemia, chronic myelogenous/myeloid leukemia (CML), myeloma, plasmacytoma, multiple myeloma (MM), plasmacytoma, myelodysplastic syndromes (MDS), chronic myelomonocytic leukemia (CMML);
- cancers of unknown primary site (CUP);

All cancers/tumors/carcinomas mentioned above which are characterized by their specific location/origin in the body are meant to include both the primary tumors and the metastatic tumors derived therefrom.

All cancers/tumors/carcinomas mentioned above may be further differentiated by their histopathological classification:
Epithelial cancers, e.g. squamous cell carcinoma (SCC) (carcinoma *in situ*, superficially invasive, verrucous carcinoma, pseudosarcoma, anaplastic, transitional cell, lymphoepithelial), adenocarcinoma (AC) (well-differentiated, mucinous, papillary, pleomorphic giant cell, ductal, small cell, signet-ring cell, spindle cell, clear cell, oat cell, colloid, adenosquamous, mucoepidermoid, adenoid cystic), mucinous cystadenocarcinoma, acinar cell carcinoma, large cell carcinoma, small cell carcinoma, neuroendocrine tumors (small cell carcinoma, paraganglioma, carcinoid); oncocytic carcinoma;

Nonepithilial cancers, *e.g.* sarcomas (fibrosarcoma, chondrosarcoma, rhabdomyosarcoma, leiomyosarcoma, hemangiosarcoma, giant cell sarcoma, lymphosarcoma, fibrous histiocytoma, liposarcoma, angiosarcoma, lymphangiosarcoma, neurofibrosarcoma), lymphoma, melanoma, germ cell tumors, hematological neoplasms, mixed and undifferentiated carcinomas;

In a further embodiment of the invention, the combinations, compositions, kits, uses, methods and compounds for use according to the invention (including all embodiments) are used to treat non-small cell lung cancer (NSCLC) (including for example locally advanced or metastatic NSCLC (stage IIIB/IV), NSCLC adenocarcinoma, NSCLC with squamous histology, NSCLC with non-squamous histology).

In a further embodiment of the invention, the combinations, compositions, kits, uses, methods and compounds for use according to the invention (including all embodiments) are used in the treatment of non-small cell lung cancer (NSCLC), in particular NSCLC adenocarcinoma.

In a further embodiment of the invention, the combinations, compositions, kits, uses, methods and compounds for use according to the invention (including all embodiments) are used in the treatment of brain cancers, in particular glioblastoma, also including brain metastasis of cancers with other origin.

In a further embodiment of the invention, the combinations, compositions, kits, uses, methods and compounds for use according to the invention (including all embodiments) are used in the treatment of soft tissue sarcoma, in particular liposarcoma.

In a further embodiment of the invention, the combinations, compositions, kits, uses, methods and compounds for use according to the invention (including all embodiments) are used in the treatment of genitourinary cancer, in particular bladder cancer.

In a further embodiment of the invention, the combinations, compositions, kits, uses, methods and compounds for use according to the invention (including all embodiments) are used in the treatment of cancer patients who are treatment naive in respect of treatment with a checkpoint inhibitor or immunomodulator, *i.e.*, *e.g.*, patients who are treatment naive in respect of treatment with a PD-1 antagonist and/or a LAG-3 antagonist or a combinaton thereof.

In a further embodiment of the invention, the combinations, compositions, kits, uses, methods and compounds for use according to the invention (including all embodiments) are used in the treatment of cancer patients who relapsed during treatment with a checkpoint inhibitor or immunomodulator, *i.e.*, *e.g.*, patients who relapsed during treatment with a PD-1 antagonist and/or LAG-3 antagonist or during treatment with a combination thereof.

In a further embodiment of the invention, the combinations, compositions, kits, uses, methods and compounds for use according to the invention (including all embodiments) are used in the treatment of a cancer which has functional p53, in particular in the treatment of a cancer with wild type p53.

The therapeutic applicability of the combination therapy according to this invention may include first line, second line, third line or further lines of treatment of patients. The cancer may be metastatic, recurrent, relapsed, resistant or refractory to one or more anti-cancer treatments. Thus, the patients may be treatment naive, or may have received one or more previous anti-cancer therapies, which have not completely cured the disease.

Patients with relapse and/or with resistance to one or more anti-cancer agents (e.g. the single components of the combination, or standard chemotherapeutics) are also amenable for combined treatment according to this invention, e.g. for second or third line treatment cycles (optionally in further combination with one or more other anti-cancer agents), *e.g.* as add-on combination or as replacement treatment.

Accordingly, some of the disclosed combination therapies of this invention are effective at treating subjects whose cancer has relapsed, or whose cancer has become drug resistant or multi-drug resistant, or whose cancer has failed one, two or more lines of mono- or combination therapy with one or more anti-cancer agents (*e.g.* the single components of the combination, or standard chemotherapeutics).

A cancer which initially responded to an anti-cancer drug can relapse and it becomes resistant to the anti-cancer drug when the anti-cancer drug is no longer effective in treating the subject with the cancer, *e.g.* despite the administration of increased dosages of the anti-cancer drug. Cancers that have developed resistance to two or more anti-cancer drugs are said to be multi-drug resistant.

Accordingly, in some methods of combination treatment of this invention, treatment with a combination according to this invention administered secondly or thirdly is begun if the patient has resistance or develops resistance to one or more agents administered initially or previously. The patient may receive only a single course of treatment with each agent or multiple courses with one, two or more agents.

In certain instances, combination therapy according to this invention may hence include initial or add-on combination, replacement or maintenance treatment.

The present invention is not to be limited in scope by the specific embodiments described herein. Various modifications of the invention in addition to those described herein may become apparent to those skilled in the art from the present disclosure. Such modifications are intended to fall within the scope of the appended claims.

All patent applications cited herein are hereby incorporated by reference in their entireties.

### Methods

### Example 1

### Anti-tumor activity of the exemplary MDM2 inhibitor BIA-1 as single agent or in combination with RMP1-14, a tool antibody to mouse PD-1, or in combination with RMP1-14 and C9B7W, a tool antibody to mouse LAG-3, in a subcutaneous syngeneic mouse model derived from the melanoma cell line B16-F10 in C57BL/6 mice.

The efficacy of the exemplary MDM2 inhibitor BIA-1 was tested in a s.c. cell line-derived syngeneic model of melanoma (B16-F10) as single agent and in combination with RMP1-14, a rat antibody to mouse PD-1 (BioXCell #BE0146), or in combination with RMP1-14 and C9B7W, a rat antibody to mouse LAG-3 (BioXCell # BE0174).

### Syngeneic mouse model

C57BL/6NTac mice were used in this study. 5 × 10⁴ melanoma cells per mouse were mixed with Matrigel and injected to establish a tumor. Tumor volume was measured two-three times per week using a caliper. Treatment started 3 days post cell injection on day 1 of the study and was stopped on day 32.

MDM2 inhibitor BIA-1 was administered daily per os (p.o.) and RMP1-14 or C9B7W were administered twice weekly i.p.. Ten animals were used in the vehicle/isotype control-treated group.

### Cells

B16-F10 cells were obtained from ATCC (CRL-6475). A master cell bank (MCB) and a working cell bank (WCB) were established. Cells were cultured in T175 tissue culture flasks at 37 °C and 5 % CO₂. The medium used was DMEM supplemented with 10 % FCS (Gibco). Cultures were detached from the plastic surface using Trypsin-EDTA in PBS (Gibco) and were split every second or third day with a ratio of 1:4/1:10.

### Mice

Mice were 7-8 week-old C57BL/6NTac purchased from Taconic, Denmark. After arrival at the animal facility, mice were allowed to adjust to ambient conditions for at least 5 days before they were used for experiments. They were housed in Macrolon^{®} type III cages in groups of ten under standardized conditions at 21.5 ± 1.5 °C and 55 ± 10 % humidity. Standardized irradiated diet (PROVIMI KLIBA) and autoclaved tap water were provided ad *libitum.* Microchips implanted subcutaneously under isoflurane anesthesia were used to identify each mouse. Cage cards showing the study number, the animal number, the compound and dose level, the administration route as well as the schedule remained with the animals throughout the study.

### Administration of test compounds

BIA-1 was suspended in 0.5 % Natrosol and administered intragastrically using a gavage needle at an application volume of 10 mL/kg per mouse once daily.

The RMP1-14 antibody and the C9B7W antibody were diluted in PBS and injected intraperitoneally with a volume of 10 mL/kg per mouse twice weekly.

### Monitoring Tumor Growth and Disease Progression

The tumor diameter was measured two-three times a week with a caliper. The volume of each tumor [in mm³] was calculated according to the formula "tumor volume = length*diameter2*π/6". To monitor side effects of treatment, mice were inspected daily for abnormalities and body weight was determined daily. Animals were sacrificed at the end of the study. Animals with necrotic tumors or tumor sizes exceeding 1500 mm³ were sacrificed early during the study for ethical reasons.

### Results

All treatment regimens were well-tolerated with reduced body weight gain but no body weight loss compared to vehicle/isotype control treated animals. Median tumor volumes were determined for each treatment group (Figure 1). As treatment benefit (response to treatment) in syngeneic mouse models is in general heterogeneous, tumor regressions at the end of the study, defined as a relative tumor volume < 1 compared day 1 of the study, were counted to assess efficacy (Figure 2).

Briefly, BIA-1 single-agent significantly delayed tumor growth but did not lead to tumor regressions at the end of the study. Neither RMP1-14 nor C9B7W single-agent significantly delayed tumor growth or led to tumor regressions at the end of the study. Both dual combinations (BIA-1 + RMP1-14 or RMP1-14 + C9B7W) significantly delayed tumor growth. While the dual combination of BIA-1 + RMP1-14 did not lead to tumor regressions at the end of the study, the dual combination of RMP1-14 + C9B7W led to 1 out 10 tumor regressions at the end of the study. The triple combination of BIA-1 + RMP1-14 + C9B7W showed best efficacy with 5 out of 10 tumor regressions at the end of the study. Taken together these data show the superior efficacy of the triple combination in an *anti*-PD-1 single-agent non-responsive model (Figure 1 and 2).

### Example 2

### Anti-tumor activity of the exemplary MDM2 inhibitor BIA-1 as single agent or in combination with RMP1-14, a tool antibody to mouse PD-1, or in combination with RMP1-14 and C9B7W, a tool antibody to mouse LAG-3, in a subcutaneous syngeneic mouse model derived from the colon cancer cell line Colon26 in Balb/C mice.

The efficacy of the exemplary MDM2 inhibitor BIA-1 was tested in a s.c. cell line-derived syngeneic model of colon cancer (Colon26) as single agent and in combination with RMP1-14, a rat antibody to mouse PD-1 (BioXCell #BE0146), or in combination with RMP1-14 and C9B7W, a rat antibody to mouse LAG-3 (BioXCell # BE0174).

### Syngeneic mouse model

BALB/cJBomTac mice were used in this study. 5 x 10⁴ colon cancer cells per mouse were mixed with Matrigel and injected to establish a tumor. Tumor volume was measured two-three times per week using a caliper. Treatment started 3 days post cell injection on day 1 of the study and was stopped on day 38.

MDM2 inhibitor BIA-1 was administered daily per os (p.o.) and RMP1-14 or C9B7W were administered twice weekly i.p.. Ten animals were used in the vehicle/isotype control-treated group.

### Cells

Colon-26 cells were obtained from Cell lines Services (CLS 400156-914SF). A master cell bank (MCB) was established. Cells were cultured in T175 tissue culture flasks at 37 °C and 5 % CO₂. The medium used was RPMI-1640 supplemented with 2mM L-Glutamine + 10 % FCS (Gibco). Cultures were detached from the plastic surface using Accutase^{®} Solution (Sigma-Aldrich) and were split between two and three times a week with a ratio of 1:4/1:6.

### Mice

Mice were 7-8 week-old BALB/cJBomTac purchased from Taconic, Denmark. After arrival at the animal facility, mice were allowed to adjust to ambient conditions for at least 5 days before they were used for experiments. They were housed in Macrolon^{®} type III cages in groups of ten under standardized conditions at 21.5 ± 1.5 °C and 55 ± 10 % humidity. Standardized irradiated diet (PROVIMI KLIBA) and autoclaved tap water were provided ad *libitum.* Microchips implanted subcutaneously under isoflurane anesthesia were used to identify each mouse. Cage cards showing the study number, the animal number, the compound and dose level, the administration route as well as the schedule remained with the animals throughout the study.

### Administration of test compounds

BIA-1 was suspended in 0.5 % Natrosol and administered intragastrically using a gavage needle at an application volume of 10 mL/kg per mouse once daily.

The RMP1-14 antibody and the C9B7W antibody were diluted in PBS and injected intraperitoneally with a volume of 10 mL/kg per mouse twice weekly.

### Monitoring Tumor Growth and Disease Progression

The tumor diameter was measured two-three times a week with a caliper. The volume of each tumor [in mm³] was calculated according to the formula "tumor volume = length*diameter2*π/6". To monitor side effects of treatment, mice were inspected daily for abnormalities and body weight was determined daily. Animals were sacrificed at the end of the study. Animals with necrotic tumors or tumor sizes exceeding 1500 mm³ were sacrificed early during the study for ethical reasons.

### Results

All treatment regimens were well-tolerated. Median tumor volumes were determined for each treatment group (Figure 3). As treatment benefit (response to treatment) in syngeneic mouse models is in general heterogeneous, tumor regressions at the end of the study, defined as a relative tumor volume < 1 compared day 1 of the study, were counted to assess efficacy (Figure 4).

Briefly, BIA-1 single-agent and RMP1-14 single-agent both led to tumor regressions at the end of the study (2/10 animals each), while C9B7W single-agent did not lead to tumor regressions at the end of the study (0/10 animals). Both dual combinations (BIA.1 + RMP1-14 or RMP1-14 + C9B7W) were superior to any of the single-agent treatments with 5/10 and 4/10 tumor regressions, respectively. The triple combination of BIA-1 + RMP1-14 + C9B7W showed best efficacy with 9/10 tumor regressions. Taken together these data show the superior efficacy of the triple combination (Figure 3 and 4).

### Example 3

### Anti-tumor activity of the exemplary MDM2 inhibitor BIA-1 as single-agent or in combination with C9B7W, a tool antibody to mouse LAG-3, or in combination with C9B7W and RMP1-14, a tool antibody to mouse PD-1, in a subcutaneous syngeneic mouse model derived from the colon cancer cell line Colon26 in Balb/C mice.

The efficacy of the exemplary MDM2 inhibitor BIA-1 was tested in a s.c. cell line-derived syngeneic model of colon cancer (Colon26) as single agent and in combination with C9B7W, a rat antibody to mouse LAG-3 (BioXCell # BE0174), or in combination with C9B7W and RMP1-14, a rat antibody to mouse PD-1 (BioXCell #BE0146).

### Syngeneic mouse model

BALB/cJBomTac mice were used in this study. 5 × 10⁴ colon cancer cells per mouse were mixed with Matrigel and injected to establish a tumor. Tumor volume was measured two-three times per week using a caliper. Treatment started 3 days post cell injection on day 1 of the study and was stopped on day 46.

MDM2 inhibitor BIA-1 was administered daily per os (p.o.) and RMP1-14 or C9B7W were administered twice weekly i.p.. Ten animals were used in the vehicle/isotype control-treated group.

### Cells

Colon-26 cells were obtained from Cell lines Services (CLS 400156-914SF). A master cell bank (MCB) was established. Cells were cultured in T175 tissue culture flasks at 37 °C and 5 % CO₂. The medium used was RPMI-1640 supplemented with 2mM L-Glutamine + 10% FCS (Gibco). Cultures were detached from the plastic surface using Accutase^{®} Solution (Sigma-Aldrich) and were split between two and three times a week with a ratio of 1:4/1:6.

### Mice

Mice were 7-8 week-old BALB/cJBomTac purchased from Taconic, Denmark. After arrival at the animal facility, mice were allowed to adjust to ambient conditions for at least 5 days before they were used for experiments. They were housed in Macrolon^{®} type III cages in groups of ten under standardized conditions at 21.5 ± 1.5 °C and 55 ± 10 % humidity. Standardized irradiated diet (PROVIMI KLIBA) and autoclaved tap water were provided ad *libitum.* Microchips implanted subcutaneously under isoflurane anesthesia were used to identify each mouse. Cage cards showing the study number, the animal number, the compound and dose level, the administration route as well as the schedule remained with the animals throughout the study.

### Administration of test compounds

BIA-1 was suspended in 0.5 % Natrosol and administered intragastrically using a gavage needle at an application volume of 10 mL/kg per mouse once daily.

The RMP1-14 antibody and the C9B7W antibody were diluted in PBS and injected intraperitoneally with a volume of 10 mL/kg per mouse twice weekly.

### Monitoring Tumor Growth and Disease Progression

The tumor diameter was measured two-three times a week with a caliper. The volume of each tumor [in mm³] was calculated according to the formula "tumor volume = length*diameter2*π/6". To monitor side effects of treatment, mice were inspected daily for abnormalities and body weight was determined daily. Animals were sacrificed at the end of the study. Animals with necrotic tumors or tumor sizes exceeding 1500 mm³ were sacrificed early during the study for ethical reasons.

### Results

All treatment regimens were well-tolerated. Median tumor volumes were determined for each treatment group (Figure 5). As treatment benefit (response to treatment) in syngeneic mouse models is in general heterogeneous, tumor regressions on day 31, defined as a relative tumor volume < 1 compared day 1 of the study, were counted to assess efficacy (Figure 6).

Briefly, BIA-1 single-agent led to tumor regressions in 4 for out 12 animals, while anti-LAG-3 single-agent did not lead to tumor regressions (0/12 animals). Efficacy of the dual combination of BIA-1+ C9B7W (4/12 tumor regressions) was comparable to single-agent BIA-1 (4/12 tumor regressions) but superior to C9B7W single-agent (0/12 tumor regressions).The triple combination of BIA-1 + RMP1-14 + C9B7W showed best efficacy with 9 out of 12 tumor regressions. Taken together these data show the superior efficacy of the triple combination (Figure 5 and 6).

### Example 4

### Anti-tumor activity of the exemplary MDM2 inhibitor BIA-1 in combination with C9B7W, a tool antibody to mouse LAG-3, and RMP1-14, a tool antibody to mouse LAG-3, compared to efficacy with RMP1-14 single-agent or efficacy of a dual combination of RMP1-14 and C9B7W in a subcutaneous syngeneic mouse model derived from the lung cancer cell line ASB-XIV in Balb/C mice.

The efficacy of the exemplary MDM2 inhibitor BIA-1 was tested in a s.c. cell line-derived syngeneic model of lung cancer (ASB-XIV) as single agent and in combination with C9B7W, a rat antibody to mouse LAG-3 (BioXCell # BE0174) and RMP1-14, a rat antibody to mouse PD-1 (BioXCell #BE0146).

### Syngeneic mouse model

BALB/cJBomTac mice were used in this study. 1 × 10⁵ lung cancer cells per mouse were mixed with Matrigel and injected to establish a tumor. Mice were pretreated twice weekly i.p. for 8 days with RMP1-14. Those mice showing no tumor growth control were randomized (= study day 1) at a tumor volume of 400 mm³ for follow-up treatments (vehicle /isotype vs. BIA1 + RMP1-14 + C9B7W vs. RMP1-14 + C9B7W vs. RMP1-14). MDM2 inhibitor BIA-1 was administered daily per os (p.o.) and RMP1-14 or C9B7W were administered twice weekly i.p. Ten animals were used in the vehicle/isotype control-treated group. Tumor volume was measured two-three times per week using a caliper. Last day of the study was day 22.

### Cells

ASB-XIV cells were obtained from Cell lines Services (CLS 400120). A master cell bank (MCB) was established. Cells were cultured in collagen coated T175 tissue culture flasks at 37 °C and 5 % CO₂. The medium used was DMEM with 4.5 g/L glucose supplemented with 1mM Sodium Pyruvate + 10% FCS (Gibco). Cultures were detached from the plastic surface using TrypleExpress (Gibco) and were split once a week with a ratio of 1:2/1:3.

### Mice

Mice were 7-8 week-old BALB/cJBomTac purchased from Taconic, Denmark. After arrival at the animal facility, mice were allowed to adjust to ambient conditions for at least 5 days before they were used for experiments. They were housed in Macrolon^{®} type III cages in groups of ten under standardized conditions at 21.5 ± 1.5 °C and 55 ± 10 % humidity. Standardized irradiated diet (PROVIMI KLIBA) and autoclaved tap water were provided ad *libitum.* Microchips implanted subcutaneously under isoflurane anesthesia were used to identify each mouse. Cage cards showing the study number, the animal number, the compound and dose level, the administration route as well as the schedule remained with the animals throughout the study.

### Administration of test compounds

BIA-1 was suspended in 0.5 % Natrosol and administered intragastrically using a gavage needle at an application volume of 10 mL/kg per mouse once daily.

The RMP1-14 antibody and the C9B7W antibody were diluted in PBS and injected intraperitoneally with a volume of 10 mL/kg per mouse twice weekly.

### Monitoring Tumor Growth and Disease Progression

The tumor diameter was measured two-three times a week with a caliper. The volume of each tumor [in mm³] was calculated according to the formula "tumor volume = length*diameter2*π/6". To monitor side effects of treatment, mice were inspected daily for abnormalities and body weight was determined daily. Animals were sacrificed at the end of the study. Animals with necrotic tumors or tumor sizes exceeding 1500 mm³ were sacrificed early during the study for ethical reasons.

### Results

Median tumor volumes were determined for each treatment group (Figure 7). Briefly, while RMP1-14 single-agent or a combination of RMP1-14 with C9B7W did not significantly delay median tumor growth, the triple combination of BIA-1 with RMP1-14 and C9B7W significantly delayed median tumor growth and led to tumor regressions.Taken together these data show the superior efficacy of the triple combination (Figure 7).

### Example 5

### Anti-tumor activity of the MDM2 inhibitor HDM-201 in combination with RMP1-14, a tool antibody to mouse PD-1, and C9B7W, a tool antibody to mouse LAG-3, in a subcutaneous syngeneic mouse model derived from the colon cancer cell line Colon26 in Balb/C mice.

The efficacy of the MDM2 inhibitor HDM-201 was tested in a s.c. cell line-derived syngeneic model of colon cancer (Colon26) in combination with C9B7W, a rat antibody to mouse LAG-3 (BioXCell # BE0174), and RMP1-14, a rat antibody to mouse PD-1 (BioXCell #BE0146).

### Syngeneic mouse model

BALB/cJBomTac mice were used in this study. 5 × 10⁴ colon cancer cells per mouse were mixed with Matrigel and injected to establish a tumor. Tumor volume was measured two-three times per week using a caliper. Treatment started 3 days post cell injection on day 1 of the study and was stopped on day 24.

MDM2 inhibitor HDM-201 was administered daily per os (p.o.) and RMP1-14 or C9B7W were administered twice weekly i.p.. Ten animals were used in the vehicle/isotype control-treated group.

### Cells

Colon-26 cells were obtained from Cell lines Services (CLS 400156-914SF). A master cell bank (MCB) was established. Cells were cultured in T175 tissue culture flasks at 37 °C and 5 % CO₂. The medium used was RPMI-1640 supplemented with 2mM L-Glutamine + 10% FCS (Gibco). Cultures were detached from the plastic surface using Accutase^{®} Solution (Sigma-Aldrich) and were split between two and three times a week with a ratio of 1:4/1:6.

### Mice

Mice were 7-8 week-old BALB/cJBomTac purchased from Taconic, Denmark. After arrival at the animal facility, mice were allowed to adjust to ambient conditions for at least 5 days before they were used for experiments. They were housed in Macrolon^{®} type III cages in groups of ten under standardized conditions at 21.5 ± 1.5 °C and 55 ± 10 % humidity. Standardized irradiated diet (PROVIMI KLIBA) and autoclaved tap water were provided ad *libitum.* Microchips implanted subcutaneously under isoflurane anesthesia were used to identify each mouse. Cage cards showing the study number, the animal number, the compound and dose level, the administration route as well as the schedule remained with the animals throughout the study.

### Administration of test compounds

HDM-201 was suspended in 0.5 % Natrosol and administered intragastrically using a gavage needle at an application volume of 10 ml/kg per mouse once daily.

The RMP1-14 antibody and the C9B7W antibody were diluted in PBS and injected intraperitoneally with a volume of 10 mL/kg per mouse twice weekly.

### Monitoring Tumor Growth and Disease Progression

The tumor diameter was measured two-three times a week with a caliper. The volume of each tumor [in mm³] was calculated according to the formula "tumor volume = length*diameter2*π/6". To monitor side effects of treatment, mice were inspected daily for abnormalities and body weight was determined daily. Animals were sacrificed at the end of the study. Animals with necrotic tumors or tumor sizes exceeding 1500 mm³ were sacrificed early during the study for ethical reasons.

### Results

Median tumor volumes were determined for each treatment group (Figure 8). The triple combination of the MDM2 inhibitor HDM-201 in combination with RMP1-14 and C9B7W led to significant tumor growth control (Figure 7). These data show that the efficacy observed with the triple combination of HDM-201 + RMP1-14 + C9B7W (Figure 8) is comparable to the efficacy observed with BIA-1 + RMP1-14 + C9B7W (Figure 3 and 5).

Examplary MDM2 inhibitor BIA-1 used in the examples is one of the compounds disclosed in Table 1.

## Claims

1. An MDM2 inhibitor for use in a method of treating and/or preventing an oncological or hyperproliferative disease, in particular cancer, said method comprising administering the MDM2 inhibitor in combination with a PD-1 antagonist to a patient in need thereof, wherein the MDM2 inhibitor is compound 28
| | |
|---|---|
| **28** | 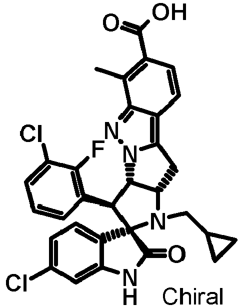 |
or a pharmaceutically acceptable salt thereof and
wherein the PD-1 antagonist is anti-PD-1 antibody PD1-3.

2. The MDM2 inhibitor for use according to claim 1, wherein the MDM2 inhibitor is administered simultaneously, concurrently, sequentially, successively, alternately or separately with the PD-1 antagonist.

3. The MDM2 inhibitor for use according to any one of claims 1 or 2, wherein the oncological disease to be treated is a cancer selected from the group consisting of lung cancer, in particular non-small cell lung cancer (NSCLC), brain cancer, in particular glioblastoma, soft tissue sarcoma, in particular liposarcoma and genitourinary cancer, in particular bladder cancer.

4. The MDM2 inhibitor for use according to any one of claims 1 to 3, wherein the cancer is non-small cell lung cancer (NSCLC), preferably non-small cell lung cancer adenocarcinoma.

5. A PD-1 antagonist for use in a method of treating and/or preventing an oncological or hyperproliferative disease, in particular cancer, said method comprising administering the PD-1 antagonist in combination with an MDM2 inhibitor to a patient in need thereof; wherein the MDM2 inhibitor is compound 28
| | |
|---|---|
| **28** | 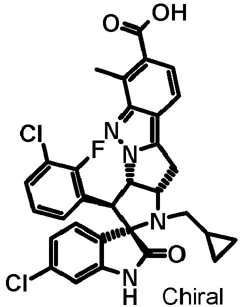 |
or a pharmaceutically acceptable salt thereof and
wherein the PD-1 antagonist is anti-PD-1 antibody PD1-3.

6. The PD-1 antagonist for use according to claim 5, wherein the PD-1 antagonist is administered simultaneously, concurrently, sequentially, successively, alternately or separately with the MDM2 inhibitor.

7. A pharmaceutical composition comprising:
• an MDM2 inhibitor,
• a PD-1 antagonist, and
• optionally, one or more pharmaceutically acceptable carriers, excipients and/or vehicles,
wherein the MDM2 inhibitor is compound 28
| | |
|---|---|
| **28** | 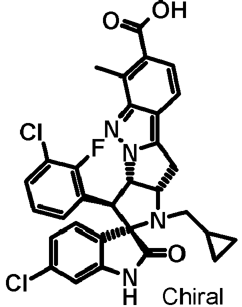 |
or a pharmaceutically acceptable salt thereof and
wherein the PD-1 antagonist is anti-PD-1 antibody PD1-3.

8. The pharmaceutical composition according to claim 7 for use in a method of treating and/or preventing an oncological or hyperproliferative disease, in particular cancer.

9. A kit comprising:
• a first pharmaceutical composition or dosage form comprising an MDM2 inhibitor and, optionally, one or more pharmaceutically acceptable carriers, excipients and/or vehicles,
• a second pharmaceutical composition or dosage form comprising a PD-1 antagonist and, optionally, one or more pharmaceutically acceptable carriers, excipients and/or vehicles,
wherein the MDM2 inhibitor is compound 28
| | |
|---|---|
| **28** | 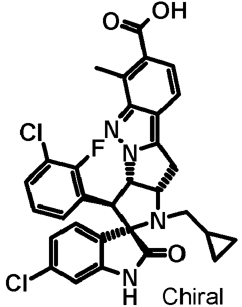 |
or a pharmaceutically acceptable salt thereof and
wherein the PD-1 antagonist is anti-PD-1 antibody PD1-3.

10. The kit according to claim 9 for use in a method of treating and/or preventing an oncological or hyperproliferative disease, in particular cancer.

11. The kit for use according to claim 10, wherein the first pharmaceutical composition or dosage form is to be administered simultaneously, concurrently, sequentially, successively, alternately or separately with the second pharmaceutical composition or dosage form.

12. The PD-1 antagonist for use according to any one of claims 5 or 6, the pharmaceutical composition for use according to claim 8, or the kit for use according to any one of claims 10 or 11, wherein the oncological disease to be treated is a cancer selected from the group consisting of lung cancer, in particular non-small cell lung cancer (NSCLC), brain cancer, in particular glioblastoma, soft tissue sarcoma, in particular liposarcoma and genitourinary cancer, in particular bladder cancer.

13. The PD-1 antagonist for use according to any one of claims 5 or 6, the pharmaceutical composition for use according to claim 8, or the kit for use according to any one of claims 10 or 11, wherein the cancer is non-small cell lung cancer (NSCLC), preferably non-small cell lung cancer adenocarcinoma.
